# FASCICULE DE BREVET EUROPEEN

(11) **EP 2 552 347 B1**
(45) Date de publication et mention de la délivrance du brevet: **12.08.2015**
(21) Numéro de dépôt: 11710229.3
(22) Date de dépôt: 28.03.2011
(51) Int. Cl.: A61K 9/00, A61D 19/00, A61K 31/01, A61K 31/02, A61K 31/12, A61K 31/37, A61K 45/06, A61K 47/10, A61K 31/121, A61K 31/035, A61K 31/131, A61K 31/201

(54) **PROCÉDÉ DE STIMULATION DES CAPACITÉS REPRODUCTIVES CHEZ LE TAUREAU ET COMPOSITION DESTINÉE À LA STIMULATION DES CAPACITÉS REPRODUCTIVES CHEZ LE TAUREAU**
VERFAHREN ZUR STIMULATION DER FORTPFLANZUNGSFÄHIGKEIT BEI EINEM BULLEN UND ZUSAMMENSETZUNG ZUR STIMULATION DER FORTPFLANZUNGSFÄHIGKEIT BEI EINEM BULLEN
METHOD FOR STIMULATING THE REPRODUCTIVE CAPACITY IN A BULL, AND COMPOSITION FOR STIMULATING THE REPRODUCTIVE CAPACITY IN A BULL

(30) Priorité: 30.03.2010 FR 1052329
(43) Date de publication de la demande: 06.02.2013
(73) Titulaire: Institut National de la Recherche Agronomique, 75007 Paris Cedex 07 (FR); Union Nationale des Coopératives Agricoles d'Elevage et d'Insémination Animale - UNCEIA, 75595 Paris Cédex 12 (FR)
(72) Inventeur: LE MEILLOUR, Patricia, F-59000 Lille (FR); LE DANVIC, Chrystelle, F-59000 Lille (FR); HUMBLOT, Patrice, F-94100 St Maur (FR); CHEMINEAU, Philippe, F-37210 Vouvray (FR); BRIANT, Christine, F-37110 Villedomer (FR); GERARD, Olivier, F-44130 Blain (FR)
(74) Mandataire: Fourcade, Emmanuelle
(86) Numéro de dépôt international: PCT/EP2011/054747
(87) Numéro de publication internationale: WO 2011/120930

(56) Documents cités:
- DE-C1- 19 808 531
- JP-A- 61 129 038
- JP-A- 2007 325 561
- SAMBRAUS H H ET AL: "THE INFLUENCE OF THE URINE OF ESTRUS COWS ON THE LIBIDO OF BULLS", ZEITSCHRIFT FUER SAEUGETIERKUNDE, vol. 40, no. 1, 1975, pages 49-54, XP009135847, ISSN: 0044-3468
- KLEMM W R ET AL: "IDENTIFICATION OF COMPOUNDS IN BOVINE CERVICO-VAGINAL MUCUS EXTRACTS THAT EVOKE MALE SEXUAL BEHAVIOR", CHEMICAL SENSES, vol. 12, no. 1, 1987, pages 77-88, XP009135893, ISSN: 0379-864X
- SANKAR R ET AL: "Detection of oestrous-related odour in bovine (Bos taurus) saliva: bioassay of identified compounds", ANIMAL, vol. 1, no. 9, octobre 2007 (2007-10), pages 1321-1327, XP002590915,
- KUMAR K RAMESH ET AL: "Chemical characterization of bovine urine with special reference to oestrus", VETERINARY RESEARCH COMMUNICATIONS, vol. 24, no. 7, novembre 2000 (2000-11), pages 445-454, XP002590604, ISSN: 0165-7380
- PALEOLOGOU A M: "Detecting oestrus in cows by a method based on bovine sex pheromones", VETERINARY RECORD, BRITISH VETERINARY ASSOCIATION, LONDON, GB, vol. 100, no. 15, 9 avril 1977 (1977-04-09), page 319, XP009135178, ISSN: 0042-4900

## Description

### DOMAINE DE L'INVENTION

L'invention concerne un procédé de stimulation des capacités reproductives chez le taureau. Plus précisément, le procédé selon l'invention permet de stimuler la fonction sexuelle, et notamment la libido, chez le taureau, mais aussi d'améliorer la qualité de la semence. L'invention concerne également une composition destinée à être utilisée sur les taureaux en vue de stimuler leurs capacités reproductives.

L'invention trouve des applications dans le domaine de l'élevage bovin, et notamment pour la récolte de la semence des taureaux, et l'insémination, directe ou artificielle, des vaches.

### ETAT DE LA TECHNIQUE ET PROBLEMES RENCONTRES

Des études récentes ont montré que le stade oestrus, correspondant à la période pendant laquelle la femelle est réceptive aux mâles et fécondable, est particulièrement brève chez la vache, de l'ordre de 6 à 12 heures. Lors de la phase oestrus, une vache sécrète des phéromones volatiles sexuelles, informant les mâles de sa disponibilité à l'accouplement. Ainsi, plusieurs études ont été conduites sur les bovins, afin de valider cette hypothèse. Ces études ont permis de confirmer la présence de telles molécules dans plusieurs sécrétions, et notamment dans les sécrétions vaginales et les urines, et leur action sur le Flehmen des mâles. Ces molécules sont détectées par les mâles et leur permettent de repérer les femelles au stade oestrus.

Actuellement, les bovins sont le plus souvent sélectionnés en fonction de caractères spécifiques, liés à la destination de l'élevage. Ainsi, les vaches de race laitière sont sélectionnées en fonction de leur capacité de lactation, devant garantir une production constante de lait, tant en qualité qu'en quantité. De même, les races à viande sont sélectionnées pour les qualités quantitatives, nutritives, gustatives, etc., de leur viande. La reproduction bovine est donc rarement laissée au hasard, les taureaux comme les vaches étant sélectionnés en fonction de critères spécifiques, liés à la destination finale de la descendance.

La collecte de la semence des taureaux en centre de production de semence est une opération délicate, devant être menée rigoureusement au niveau de la collecte elle-même, de manière notamment à éviter les contaminations, mais également lors des manipulations des taureaux, en amont et en aval de la collecte.

Actuellement, il n'est pas possible de mettre au point un protocole commun à tous les taureaux d'un élevage, pour garantir une collecte à peu près uniforme entre tous les individus de l'élevage. En effet, au sein d'un même élevage, les individus peuvent présenter une libido variable, tant d'un individu à un autre que, pour un même individu, d'un moment à un autre.

Le document de Sambraus et al. (Zeitschrift Fuer Saeugetierkunde, vol. 40, no. 1, 1975, pages 49-54) décrit un test d'évaluation de l'effet de l'urine de vache sur le comportement sexuel des taureaux.

Le document de Sankar et al. (Animal, vol. 1, no. 9, octobre 2007, pages 1321-1327) décrit des composants présents dans la salive de la vache lors de la phase oestrale et impliqués dans l'attirance du mâle : la triméthylamine, l'acide acétique, le 4-propyl phénol, l'acide pentanoïque et l'acide propionique.

Le document de Paleologou (Veterinary record, vol. 100, no. 15, 9 avril 1977, page 319) décrit l'effet du mucu cervico-vaginal de la vache sur le comportement sexuel du taureau.

Le document JP 2007 325561 décrit quant à lui une composition comprenant du coumarin, pour stimuler l'appétit chez les herbivores. Il ne précise nullement de concentration de ce composé dans la composition, et ne divulgue pas non plus l'utilisation de cette composition pour améliorer la fonction reproductive chez le taureau.

A ce jour, il n'existe aucune méthode efficace pour stimuler la fonction sexuelle et tout particulièrement la libido des taureaux utilisés en centre d'insémination ou en élevage. Or, les troubles de la libido sont extrêmement répandus chez les taureaux et s'avèrent pénalisants pour les centres de sélection de semence. Ils peuvent se traduire par des pertes économiques non négligeables (pertes de doses, pertes de temps importantes, risques pour les bouviers). Le temps et les conditions de collecte sont donc des paramètres difficilement prévisibles pour les bouviers.

De même, la qualité et la quantité de la semence sont fluctuantes d'un individu à un autre, et pour un même individu d'un moment à un autre. A ce jour, il n'existe pas de procédé efficace pour améliorer la production de la semence chez les taureaux, sinon la gestion individualisée des collectes, prenant en compte le comportement de chaque individu.

### EXPOSE DE L'INVENTION

L'invention a pour but de résoudre tout ou partie des problèmes exposés ci-dessus. Notamment, un des buts de l'invention est d'améliorer les capacités reproductives des taureaux, afin de récupérer leur semence en vue d'inséminations ultérieures et/ou afin de procéder à un accouplement avec des femelles au stade oestrus. Les capacités reproductives s'entendent des capacités comportementales, et notamment la libido, et/ou des capacités physiologiques, et notamment la qualité et/ou la quantité de semence.

Pour cela, l'invention propose d'utiliser certaines molécules, isolées initialement à partir de certaines sécrétions de vache au stade oestrus, et notamment dans les urines, pour les soumettre aux taureaux avant collecte de leur semence ou avant de les présenter à des femelles en vue de la reproduction. Les inventeurs ont notamment identifié six molécules spécifiques du stade oestrus et ayant des effets sur la capacité reproductive des taureaux, à savoir le coumarin, le squalène, le 6-aminoundécane, la 2-butanone, l'acide 9-octadécénoïque et le 1,2-dichloroéthylène. Ces molécules peuvent être isolées à partir des sécrétions de vache au stade oestrus, ou être obtenues par tout autre moyen et notamment par synthèse chimique. Certaines de ces molécules sont d'ailleurs déjà disponibles dans le commerce.

Les molécules selon l'invention sont des phéromones incitatrices, c'est-à-dire qui agissent sur le comportement, mais également modificatrices, c'est-à-dire qui agissent sur la biologie. Plus particulièrement, l'inhalation de ces molécules permet de réduire le temps avant la monte et l'éjaculation, et/ou d'augmenter la concentration en spermatozoïdes dans l'éjaculat.

Les utilisations qui en sont faites permettent, avec une gestion environnementale équivalente de la collecte, d'augmenter la production de semence de l'ensemble des individus, avec des effets d'interaction traitement/individu négligeables. Cette amélioration de la production de semence est tout à fait intéressante sur le plan économique, en particulier pour les individus de grande valeur génétique. De plus, l'obtention d'effets similaires chez les animaux jeunes est très intéressante, notamment dans le cadre de la sélection génomique, où l'on cherche à optimiser les performances reproductives de tels individus pour accélérer le progrès génétique.

L'invention propose tout particulièrement d'utiliser ces molécules en solution apte à être pulvérisée dans les naseaux des taureaux à traiter. En effet, de cette façon, les molécules sont directement en contact avec la cible, et les risques que les molécules, très volatiles, se dissipent dans l'air sont réduits. Par ailleurs, ce mode d'administration permet de doser de manière précise la quantité de phéromones inhalée par individu.

### BREVE DESCRIPTION DES FIGURES

La figure 1 montre les profils chromatographiques comparés de la composition chimique des urines de vache au cours d'un cycle oestral ;
Les figures 2A à 2F montrent les spectres ioniques expérimentaux (a), en comparaison avec les spectres de la banque NIST (b), de six composés chimiques spécifiques des stades pré-oestrus et oestrus, identifiés selon l'invention, dans les urines et mucus vaginaux des vaches ;
Les figures 3A à 3C montrent les effets d'un premier exemple de traitement selon l'invention sur la libido (figure 3A), la concentration en spermatozoïdes des éjaculats en milliards de spermatozoïdes / mL (figure 3B) et sur le nombre total de spermatozoïdes en milliards, par éjaculat (figure 3C). ;
Les figures 4A et 4B montrent les effets du premier exemple de traitement selon l'invention sur la semence d'un groupe de taureaux à libido normale (concentration en spermatozoïdes : figure 4A ; nombre total de spermatozoïdes : figure 4B) ;
La figure 5 montre les effets du premier exemple de traitement selon l'invention sur la libido d'un groupe de taureaux à faible libido initiale ;
Les figures 6A et 6B montrent les effets d'un second exemple de traitement selon l'invention sur la libido (figure 6A) et sur la semence des taureaux (figure 6B), en fonction de leur libido initiale.

### DESCRIPTION DETAILLEE DE L'INVENTION

Les inventeurs ont identifié des molécules volatiles, présentes spécifiquement dans certaines sécrétions des vaches au stade oestrus, et qui ont une action bénéfique sur la fonction reproductive des taureaux, en ce sens qu'elles sont aptes à améliorer et/ou induire la libido des taureaux, et/ou à améliorer la qualité et/ou la quantité de semence produite.

Selon l'invention, le comportement sexuel, ou la libido, des taureaux prend en compte le temps de réaction, le temps de préparation sexuelle et le temps jusqu'à l'éjaculation. Le temps de réaction est défini selon l'invention comme l'intervalle entre le moment où le taureau est amené à environ 1,5m du boute-en-train et la première fausse monte. Le temps de préparation sexuelle correspond à la préparation préconisée par Amann (1976), à savoir : une fausse monte, suivie de 2 minutes d'attente active, puis deux autres fausses montes au gré du taureau, enfin, sans autre attente, la monte conduisant à l'éjaculation. L'attente active se définit par le maintien du taureau au contact du boute-en-train, les deux animaux se trouvant côte à côte. Le temps jusqu'à l'éjaculation est, lui, égal à la somme du temps de réaction et du temps de préparation sexuelle.

L'invention a donc pour objet l'utilisation d'une composition comportant au moins une molécule volatile spécifique de l'oestrus chez la vache pour améliorer la fonction reproductive chez le taureau, telle que définie dans la revendication 1.

Par vache, il faut entendre la femelle de l'espèce bovine, en âge de procréer, c'est-à-dire le plus souvent ayant plus de 18 mois. De même, par taureau, il faut entendre le mâle de l'espèce bovine, non castré et en âge de procréer.

L'oestrus correspond à la période du cycle oestral, également appelée chaleurs, où a lieu l'ovulation et où la femelle accepte l'accouplement. L'oestrus est précédé par la phase pré-oestrus, et est suivi de la phase post-oestrus.

Par fonction reproductive, on entend les processus comportementaux et physiologiques aboutissant à la production de semence. Selon l'invention, l'amélioration de la fonction reproductive peut consister en une amélioration de la libido, c'est-à-dire une amélioration du comportement conduisant à la production de la semence, mais également en une amélioration de la semence elle-même, ou les deux.

Par amélioration, on entend un accroissement de la capacité reproductive du taureau, que se soit par réduction du temps nécessaire audit taureau pour produire la semence et/ou par augmentation de la qualité intrinsèque de la semence elle-même. Notamment, la qualité de la semence peut être améliorée par augmentation du volume produit, par augmentation de la concentration en spermatozoïdes dans ledit volume, par augmentation du nombre de spermatozoïdes motiles, etc.

Selon l'invention, l'utilisation de cette composition permet d'améliorer les performances de collecte de la semence du taureau traité, ou d'augmenter les chances d'obtenir une fécondation suite à la saillie par le taureau traité d'une vache en chaleur.

Dans la composition utilisée, au moins une molécule volatile spécifique du stade oestrus chez la vache est choisie parmi le coumarin, le squalène, le 6-aminoundécane, la 2-butanone, l'acide 9-octadécénoïque et le 1,2-dichloroéthylène. Avantageusement, la composition contient au moins un mélange de coumarin et de squalène.

Dans d'autres modes de mise en oeuvre préférés de l'invention, la composition comporte au moins un mélange de squalène et de 1,2-dichloroéthylène. Toute combinaison de deux ou plus des molécules volatiles spécifiques du stade oestrus chez la vache ci-dessus entre également dans le cadre de l'invention.

La composition selon l'invention est avantageusement conditionnée sous forme de solution comportant du glycérol en tant que solvant des molécules volatiles.

Préférentiellement, la concentration totale en molécules volatiles spécifiques du stade oestrus est comprise entre 2,5 pg/mL et 500 pg/mL. En particulier, elle est comprise entre 5 pg/mL et 100 pg/mL, ou entre 2,5 pg/mL et 5 pg/mL, ou encore entre 100 pg/mL et 500 pg/mL. Par concentration totale, on entend la somme des concentrations en chacune des molécules volatiles spécifiques du stade oestrus utilisées pour réaliser la composition. De manière encore préférée, la concentration en molécules volatiles spécifiques de l'oestrus dans la composition est au moins égale à 25 pg/mL, +/- 5%.

Dans d'autres modes de mise en oeuvre préférés de l'invention, la concentration en chaque molécule volatile spécifique du stade oestrus chez la vache contenue dans la composition est supérieure ou égale à 2,5 pg/mL.

Selon des exemples d'utilisation de la composition selon l'invention, il est possible de maintenir ladite composition à la périphérie des naseaux du taureau de manière à ce que ledit taureau puisse inhaler ladite composition.

Préférentiellement, la composition est conditionnée sous forme de spray de manière à pouvoir être pulvérisée directement dans les naseaux du taureau.

Selon l'invention, la composition peut avantageusement être utilisée avec effet immédiat. Autrement dit, il n'y a pas de temps de latence pour que la composition agisse, et il est possible de procéder à la saillie d'une femelle ou à la récolte de la semence directement après avoir soumis le taureau à ladite composition.

Avantageusement, on soumet le taureau à ladite composition avant chaque récolte de semence ou avant chaque saillie d'une vache.

L'invention concerne également une composition destinée à être utilisée sur un taureau, afin d'améliorer sa fonction reproductive, telle que définie dans la revendication 13.

Avantageusement, dans la composition, au moins une molécule volatile spécifique de l'oestrus chez la vache est choisie parmi le coumarin, le squalène, le 6-aminoundécane, le 2-butanone, l'acide 9-octadécénoïque et le 1,2-dichloroéthylène.

Dans des modes de réalisation préférés de l'invention, la composition comporte au moins un mélange de squalène et de 1,2-dichloroéthylène. Toute combinaison de deux ou plus des molécules volatiles spécifiques du stade oestrus chez la vache ci-dessus entre également dans le cadre de l'invention.

La composition peut être conditionnée sous forme de solution contenant préférentiellement du glycérol en tant que solvant des molécules volatiles. En effet, le glycérol n'est pas nocif pour l'animal et n'agit pas lui-même sur la fonction reproductive du taureau. Bien entendu, tout autre solvant ou excipient, apte à diluer les molécules selon l'invention, et permettant une utilisation sans risque sur le taureau, peut être utilisée.

Préférentiellement, la solution comporte les excipients nécessaires pour permettre sa pulvérisation.

Selon l'invention, la solution présente avantageusement une concentration totale en molécules volatiles spécifiques du stade oestrus comprise entre 2,5 pg/mL et 500 pg/mL. En particulier, elle est comprise entre 5 pg/mL et 100 pg/mL, ou entre 2,5 pg/mL et 5 pg/mL, ou encore entre 100 pg/mL et 500 pg/mL. De manière encore préférée, la concentration en molécules volatiles spécifiques de l'oestrus dans la composition est au moins égale à 25 pg/mL, +/- 5%.

Dans d'autres modes de réalisation préférés de l'invention, la concentration en chaque molécule volatile spécifique du stade oestrus chez la vache contenue dans la composition est supérieure ou égale à 2,5 pg/mL.

### EXPERIMENTATIONS

### I- MATERIEL ET METHODES

### 1 - Collectes des échantillons biologiques (urines et mucus vaginaux)

Afin de mettre en évidence les composés chimiques impliqués dans la détection de l'oestrus par le mâle chez les bovins, plusieurs collectes d'urine et de mucus vaginaux ont été réalisées sur des vaches de réforme ou des génisses.

Dans un premier temps, deux séries de collectes ont été réalisées sur des vaches de réforme de race Prim'Holstein.

Les urines ont été collectées sur des animaux synchronisés par traitement à la prostaglandine (PGF2α) au moment des mictions naturelles à trois stades du cycle, à savoir en pré-cestrus (J+1 post-PGF2α), en oestrus (J+2 post-PGF2α) et en phase lutéale (J+7 post-PGF2α). Un suivi des cycles au cours de ces collectes a été réalisé, de façon à déterminer au mieux le moment de l'oestrus, par observation journalière des animaux, associée à un dosage de la progestérone plasmatique.

Des mucus vaginaux ont également été collectés par tamponnage d'une gaze sur la zone péri-vaginale lors de l'apparition de mucus vaginal.

Une session de collecte a également été réalisée sur des génisses de 18 mois, au cours de leurs premiers cycles oestraux. Les échantillons urinaires ont été collectés en pré-oestrus (entre J-3 et J-1 avant l'oestrus), en oestrus et en phase lutéale (J+10) au cours de cycles naturels. La détermination des stades a été réalisée par un suivi minutieux des comportements à raison de 5 observations visuelles par jour pendant 15 min sur plus de 36 mois. Des échographies, à raison de 3 fois par semaine, y ont été associées de façon à surveiller la maturation folliculaire.

Trois autres sessions de collectes ont été réalisées de la manière suivante :
Six à huit vaches (races Holstein et Montbéliarde) ont été synchronisées dans leur cycle par deux injections de Prostaglandine (PGF2α) à 10 jours d'intervalle, avec une seconde injection réalisée le jour des premières collectes (J0). Des dosages plasmatiques hormonaux ont été réalisés en complément des observations visuelles et comportementales, afin de déterminer le moment de l'ovulation. Des prises de sang ont donc été effectuées toutes les 6 heures afin de doser l'hormone lutéinisante (LH) et l'oestradiol (E2) durant la semaine ayant suivi la seconde injection de prostaglandine. Les dosages de progestérone (P4) ont été effectués toutes les 24 heures. Le sérum issu de chaque prise de sang a été stocké à -80°C jusqu'au dosage de la progestérone (kit ovucheck plasma, Biovet), de l'hormone lutéinisante (kit LH DETECT, INRA de Nouzilly) et de l'oestradiol. Un suivi du développement folliculaire a été réalisé en complément, lors de l'une des collectes à raison de deux échographies par jour.

Les urines ont été collectées lors de mictions spontanées à raison de deux à trois fois par jour en fonction des animaux. Au moment de la collecte, les urines ont été fractionnées dans 4 tubes de 4 ml en verre scellés par des bouchons en téflon (Supelco).

Les mucus cervicaux, fluides biologiques spécifiques des chaleurs, ont également été collectés dans des flacons de 20 ml en verre fermés par des bouchons en téflon (Supelco).

Dans tous les cas, les échantillons collectés ont été conservés à 4°C après collecte avant un stockage dans les 4 heures à -80°C jusqu'à leur analyse.

### 2 - Analyse de la composition chimique des fluides biologiques échantillonnés

### 2 - 1 - Extraction par solvant organique

L'extraction chimique a été effectuée volume à volume par un solvant organique à partir de 4 mL d'urine ou de mucus vaginaux décongelés sur glace.

Plusieurs solvants ont été testés (dichlorométhane=DCM, pentane, hexane). Le DCM, qui présentait une capacité d'extraction d'un plus grand nombre de composés chimiques sur les premiers échantillons analysés, a été retenu. Les extractions se sont faites dans des tubes en verre fermés par des bouchons à vis en téflon (Supelco) afin de minimiser les contaminations par des résidus plastiques (les phtalates).

Après passage rapide au vortex, les échantillons ont sédimenté à température ambiante pendant 10 min, avant d'être centrifugés à 2500 rpm durant 20 min. Après cette centrifugation, la phase organique a été séchée sur colonne de sulfate de sodium (FLUKA) avant d'être concentrée 100 fois sous flux d'azote et stockée à -20°C jusqu'aux analyses (dans les deux semaines suivant l'extraction).

### 2 - 2 - Identification des composés chimiques par GC/MS

Les extraits chimiques ont été analysés par chromatographie en phase gazeuse, couplée à une spectrométrie de masse (GC-MS) sur un appareil de type FOCUS/DSQII (Thermo Scientific) constitué d'un Focus GC couplé à un spectromètre de masse de type quadrupole (DSQII, Thermo Scientific).

Au cours des analyses (réalisées sur 3 ans), cinq colonnes différentes ont été utilisées pour séparer les composés extraits : une CP-sil5 CB low Bleed/MS 60 m x 0,25 µm ID x 0,25 µm (SUPELCO), une TR-1 MS 30 m x 0,25 mm ID x 0,25 µm (Thermo), une 1MS-SolGel 30 m x 0,25 µm ID x 0, 25 µm (SGE), une TR-1MS 30m x 0,25 ID x 0,25 m (Thermo), une TR-1MS 30m x 0,25 ID x 0,25 m (Thermo) et une Equity 5 30 m x 0,25 µm ID x 0, 25 µm (SUPELCO).

Des aliquots de 2 µl d'extrait ont été injectés en mode splitless (30 sec) dans un injecteur à 260°C.

Le programme d'analyse utilisé a été le suivant : 30°C pendant 4 min puis 5°C/min jusqu'à 280°C, et isotherme à 280°C pendant 10 min.

L'ionisation des molécules entrant dans le spectromètre de masse a été obtenue par impact électronique à 70 eV, avec une température de source de 180°C.

L'identification des composés d'intérêt a été effectuée par comparaison des spectres ioniques expérimentaux obtenus avec la banque de spectres NIST.

L'analyse a été focalisée sur les molécules variant au cours du cycle oestral de l'animal et plus particulièrement aux cours des stades pré-oestrus et oestrus.

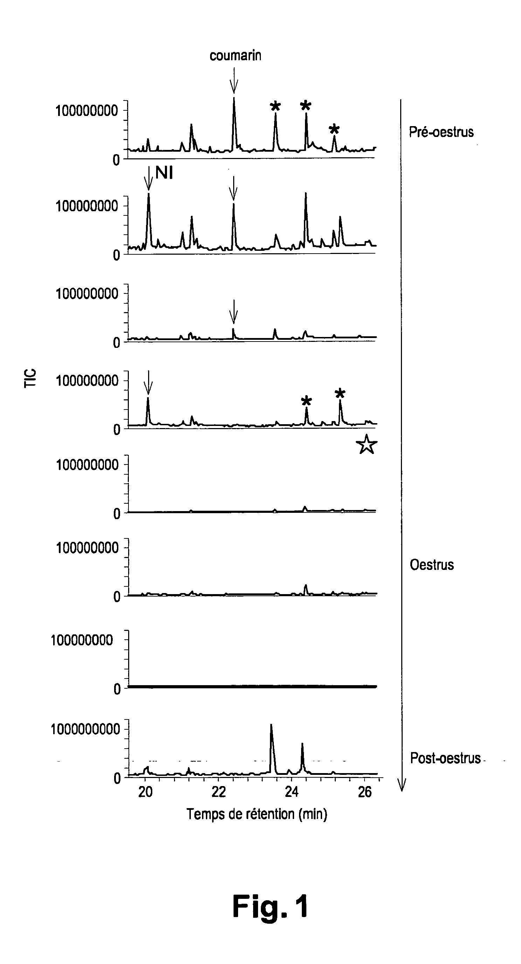

La figure 1 illustre la façon dont les molécules d'intérêt ont été mises en évidence. Les différents stades du cycle sont représentés sur la droite des chromatogrammes par une échelle de temps. Une étoile indique le moment de l'ovulation. Les pics surmontés d'un astérisque correspondent à des composés non spécifiques de stade, car retrouvés chez d'autre animaux en phase post-oestrus, et donc non retenus. Les pics surmontés d'une flèche correspondent à des pics spécifiques des stades pré-oestrus/oestrus chez une majorité des animaux, que les composés aient été identifiés (exemple : le coumarin à la figure 1) ou non identifiés (exemple : le pic surmonté de « NI » à la figure 1).

### II- RESULTATS

### 1- Identification des molécules

Une dizaine de molécules a été mise en évidence comme étant de potentiels marqueurs de l'oestrus en raison de leur présence aux stades pré-oestrus et/ou oestrus chez une majorité de vaches.

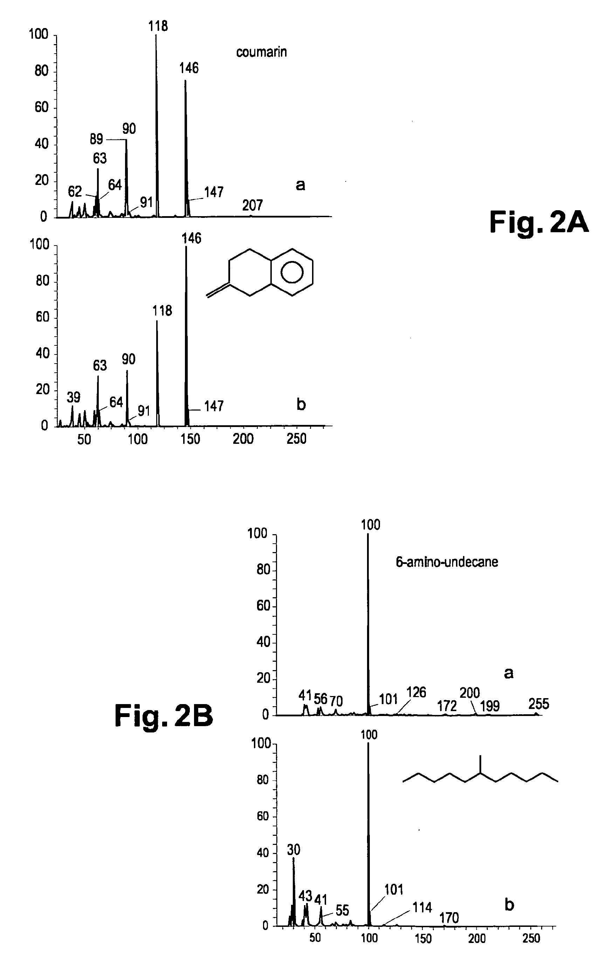

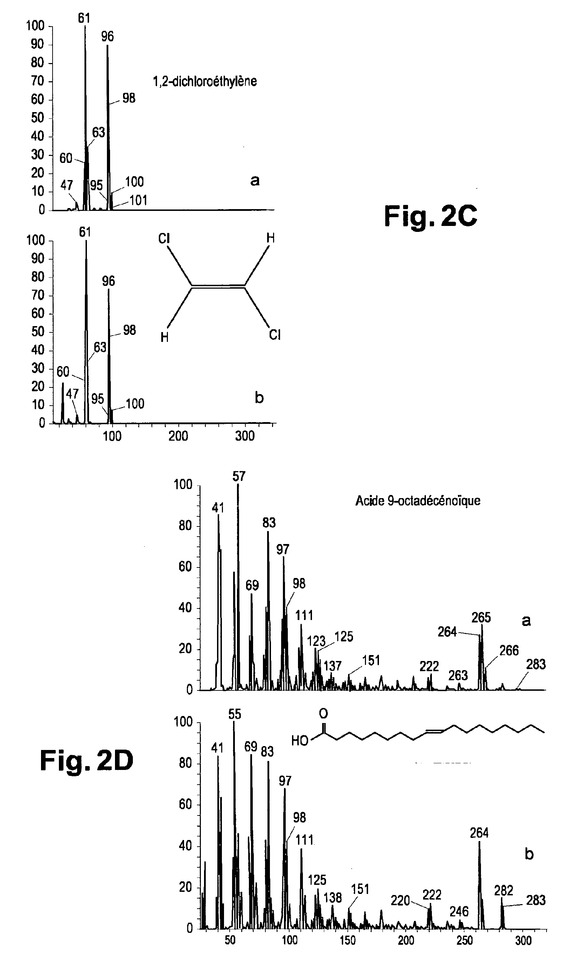

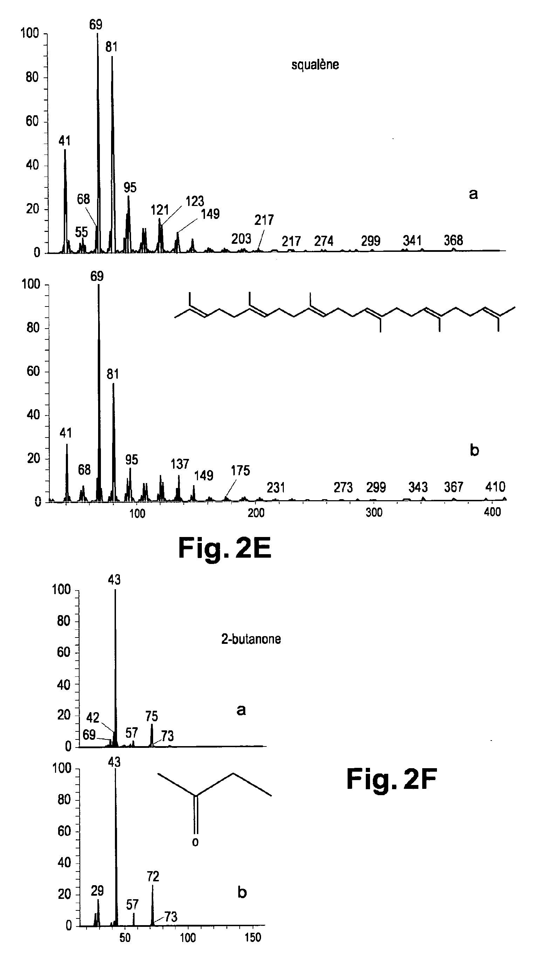

Six de ces molécules, le coumarin, le 6-aminoundécane, le 1,2-dichloroéthylène, le squalène, la 2-butanone et l'acide 9-octadécénoïque, ont pu être identifiées par comparaison de leur spectre ionique (expérimental) avec ceux de la banque de spectre NIST. Les figures 2A, 2B, 2C, 2D, 2E et 2F comparent les spectres ioniques expérimentaux (a) de ces molécules avec les spectres de la banque NIST (b).

Ainsi, le coumarin était présent chez 5 des 6 vaches aux stades pré-oestrus et/ou oestrus dans les urines de vache de la deuxième collecte. Lors de cette collecte, il a été mis en évidence en association avec d'autres molécules, qui ne sont pas toutes identifiées actuellement. Toutefois, le 6-aminoundécane a pu être identifié en pré-oestrus chez 5 des six vaches analysées et en oestrus chez 2 d'entre elles. Le coumarin a également été retrouvé au stade oestrus chez une vache de la première collecte.

Le 1,2-dichloroéthylène a été mis en évidence lors de l'analyse de la troisième collecte réalisée au stade pré-oestrus chez la moitié des vaches étudiées et pour trois vaches sur huit en oestrus. Sa présence a également pu être détectée dans les urines de génisse au cours de leurs premières chaleurs.

Le squalène est la molécule identifiée qui a été retrouvée le plus régulièrement aux stades pré-oestrus/oestrus quelle que soit la collecte. De façon intéressante, on a observé cette molécule à la fois dans les urines aux stades définis précédemment, et dans les mucus vaginaux, fluides biologiques spécifiques de l'oestrus chez la vache.

L'acide 9-octadécénoïque est un acide gras qui a également été retrouvé à la fois dans certaines urines (bien que moins fréquemment que le squalène) aux stades désirés et dans les mucus vaginaux.

La 2-butanone a également été identifiée de façon quasi-systématique dans les mucus vaginaux des vaches collectées.

### 2- Préparation de compositions et conditionnements

Trois molécules, disponibles commercialement, ont été utilisées pour préparer les compositions utilisées dans les exemples ci-dessous, à savoir le coumarin, le squalène et le 1,2-dichloroéthylène.

Pour la réalisation des compositions, l'excipient employé est une solution de glycérol/eau (50/50, v/v). Ce même excipient est utilisé comme solution témoin (Solution T).

### 2-1- Solution A

La solution A contient le coumarin à une concentration finale de 25 pg/ml. Une première dilution du coumarin commercial a été réalisée dans de l'éthanol pour une concentration finale de 12,5 mg/ml, puis deux dilutions au 100^{ème} ont été réalisées en série dans une solution d'éthanol pour arriver à une solution mère A à une concentration de 12,5 ng/ml. Un millilitre de cette dernière dilution est dilué dans 499 ml d'excipient pour obtenir la solution A finale.

### 2-2- Solution B

La solution B contient un mélange de squalène et de 1,2-dichloroéthylène à une concentration totale de 25 pg/ml. De la même façon que précédemment, 12,5 mg des composés commerciaux ont été repris dans 1 ml d'éthanol pour obtenir une solution mère B à 12,5 mg/ml. Ces solutions mères ont été diluées au 1000^{ème} en série 23 fois dans de l'éthanol pour arriver à une concentration de 12,5 ng/ml avant dilution finale dans l'excipient pour obtenir la solution B finale.

### 2-3- Solution A+B

La solution A+B est obtenue par dilution de 1 ml des solutions mères A et B (à 12,5 ng/ml) dans 498 ml d'excipient.

### 2-4- Conditionnement

Les solutions ont été conditionnées sous forme de spray, dans des pulvérisateurs utilisés en cosmétologie, en aluminium doublé d'un revêtement neutre utilisé dans l'industrie agro-alimentaire pour éviter le relarguage de substances parasites. Un tel conditionnement permet de soumettre le taureau à la composition en limitant les pertes en molécules actives, c'est-à-dire les phéromones d'intérêt, qui sont par nature très volatiles. En effet, le dépôt peut se faire directement dans les naseaux de l'animal, donc proche de la cible, à savoir le système nerveux. L'utilisation d'un pulvérisateur permet donc, par une méthode non invasive, de limiter la perte en molécules actives, de contrôler la dose fournie à l'animal, et d'être au plus proche de la cible.

Autrement, il est également possible de présenter la solution dans des coupelles que l'animal reniflera et/ou léchera. Il est également possible d'utiliser la solution pour badigeonner les parties génitales, entre autres, de la vache qui doit être saillie, ou le boute-en-train utilisé dans le cadre de la récolte de la semence du taureau.

### EXEMPLE I

### I- MATERIEL ET METHODES

### 1- Matériel animal

Un lot de dix taureaux adultes, âgés de 4 ans à 5,5 ans, de races Prim Holstein ou Normande, a été inclus dans la première étude. Ces animaux ont été recrutés dans la filière « insémination animale » dès l'âge de 2 à 3 mois et depuis n'ont jamais été en contact avec des femelles bovines. Ils ont toujours été élevés en boxes individuels (période de prétestage ou période de production) ou en lots de 4 à 6 animaux de même âge et de même sexe (période de lay-off). Les conditions d'alimentation et d'hébergement ont toujours été identiques pour les animaux de l'essai, avant et pendant l'essai.

Préalablement à l'essai, les taureaux ont été répartis en 2 groupes en fonction de leur libido, définie à partir de leur comportement observé avant l'essai en salle de monte. Le groupe 1 correspond aux animaux à libido « normale », c'est-à-dire dont les temps de réaction et d'éjaculation sont conformes à ceux de l'ensemble des taureaux de la taurellerie. Le groupe 2, à « faible libido », est constitué des reproducteurs considérés comme « lents » voire « très lents » jusqu'à la manifestation d'un intérêt pour le boute-en-train et l'obtention d'un éjaculat.

### 2- Solution utilisée

Dans cet exemple I, la solution B a été testée.

La solution contenant les molécules retenues (Solution B dans le tableau 1) a été conditionnée sous forme de spray expérimental, dans des pulvérisateurs.

Une version témoin (solution T dans le tableau 1) a été réalisée, en utilisant uniquement l'excipient employé également pour la mise en solution des molécules actives de la solution B.

Les pulvérisateurs portaient des numéros de code (1, 2) dont le sens était inconnu des taureliers, sauf de la personne chargée d'administrer le traitement en début de collecte.

### 3- Organisation générale des essais : distribution des solutions

Le tableau 1 ci-dessous reprend pour chacun des groupes de taureaux les expérimentations réalisées.

Les essais ont duré au total 6 semaines. Une seule personne, toujours la même, était habilitée à pulvériser les solutions dans les naseaux des taureaux.

Les deux premières semaines ont été consacrées à l'entraînement des taureaux au rythme de collecte, à la stabilisation des réserves spermatiques et à l'estimation du potentiel de production des individus. Elles ont permis de s'assurer de la pertinence de la répartition entre les 2 lots : différence significative de libido estimée par les temps de réaction et d'éjaculation et absence de différence entre paramètres de production.

Les quatre semaines suivantes ont constitué la phase expérimentale proprement dite, et ont été programmées en carré latin : chaque lot a subi alternativement la distribution du témoin et du traitement et chaque taureau a été également son propre témoin.

Pendant les deux semaines d'adaptation, les taureaux ont reçu uniquement le spray témoin contenant l'excipient, à raison d'une pulvérisation par naseau. Ensuite et en fonction de leur attribution dans le protocole expérimental, les animaux ont reçu soit le spray témoin soit un spray expérimental.

Les molécules utilisées étant très volatiles, le lot « témoin » a toujours commencé la récolte, afin de ne pas saturer l'atmosphère de la salle de collecte en molécules actives. Tous les animaux ont reçu le traitement à leur arrivée en salle de monte, qui s'est fait de façon aléatoire au sein de chaque groupe.

**Tableau 1 : Organisation générale des essais pour les taureaux adultes**

| | Semaine 1 | Semaine 2 | Semaine 3 | Semaine 4 | Semaine 5 | Semaine 6 |
|---|---|---|---|---|---|---|
| Groupe 1 | Solution T | Solution T | Solution T | Solution B | Solution T | Solution B |
| Libido normale | Adaptation | Adaptation | Expérimentation | Expérimentation | Expérimentation | Expérimentation |
| Groupe 2 | Solution T | Solution T | Solution B | Solution T | Solution B | Solution T |
| Faible libido | Adaptation | Adaptation | Expérimentation | Expérimentation | Expérimentation | Expérimentation |

| | | | | | | |
|---|---|---|---|---|---|---|
| Solution T : solution témoin, ne contenant que le solvant Solution B : solution contenant les phéromones selon l'invention et le solvant | | | | | | |

### 4- Enregistrement des paramètres comportementaux

Les taureaux des essais ont été soumis, pendant tous les essais, à un rythme hebdomadaire de 3 collectes d'un éjaculat : lundi, mercredi, vendredi.

La préparation des taureaux s'est faite systématiquement, en respectant le schéma décrit par Amann (voir définition ci-dessus).

Pour évaluer la libido, il faut donc procéder à l'enregistrement, avant la collecte de chaque éjaculat, de ces trois temps, le temps de réaction et le temps jusqu'à l'éjaculation étant mesurés au chronomètre.

Le boute-en-train est resté le même au cours d'une même séance de collecte. Toutefois, si au bout de 10 minutes un taureau n'avait manifesté aucune réaction, le boute-en-train a pu être changé.

Le chef d'équipe a été le seul habilité à pulvériser les traitements en fonction de l'appartenance des animaux à chacun des groupes et à chronométrer les différents temps.

Paramètres de libido : Les temps de réaction (Treact) et d'éjaculation (Tejac) précédemment décrits ont été mesurés (en secondes) à chaque saut pour chaque taureau. Les manifestations de Flehmen, de pompage et les émissions de jets pré-spermatiques ont été enregistrées.

Sur la durée de l'essai, trois personnes ont chronométré les différents temps.

De même, la qualité du saut a été notée : absence, faible, moyen, franc, très franc.

Pour les autres aspects, les collectes se sont déroulées dans les conditions de routine en vigueur au sein de la taurellerie.

### 5- Évaluation des paramètres biologiques

Une fois collectée à l'aide d'un vagin artificiel, la semence a été transmise directement au laboratoire pour être traitée dans les conditions habituelles de travail.

Les paramètres biologiques quantitatifs, à savoir le volume (mL), la concentration (milliards de spz/ml), et le nombre total de spermatozoïdes par éjaculat (en milliards) et qualitatifs, à savoir le % de spermatozoïdes vivants et la qualité de mouvement, ou motilité, ont été enregistrés pour chaque éjaculat collecté.

### 6- Analyse statistique

Une comparaison de chacun des groupes de taureaux, à savoir Groupes 1 et 2, pendant la période d'adaptation a été réalisée afin de s'assurer que les seules différences concernent la libido des animaux.

Ensuite, une première analyse de variance Proc GLM (avec et sans semaines d'adaptation) sur la population globale a été effectuée avec le logiciel SAS et a porté sur :
- les paramètres de libido, à savoir le temps de réaction et le temps jusqu'à l'éjaculation,
- les paramètres biologiques, à savoir le volume, la concentration, le nombre total de spermatozoïdes, le pourcentage de spermatozoïdes vivants, la qualité du mouvement.

Les facteurs de variation pris en compte dans le modèle ont été les suivants : Période, Semaine, Taureau, Traitement, Interactions 2 à 2.

Une seconde analyse Proc GLM intra groupe, sans les semaines d'adaptation, a été menée afin de voir si les effets du traitement étaient similaires en fonction de la libido initiale des taureaux. Enfin, les effets du mélange sur les mâles pris individuellement ont été analysés.

### II- RESULTATS

### 1- Comparaison initiale des lots de taureaux (avant traitement)

Les performances des 2 lots de taureaux (G1 : libido normale, G2 : faible libido) ont été comparées à partir de l'ensemble des paramètres enregistrés pendant la période d'adaptation. L'observation a porté sur 6 collectes, soit un total de 30 éjaculats par groupe. Les résultats obtenus sont répertoriés dans le tableau 2 ci-dessous.

**Tableau 2 : comparaison des moyennes et écarts-types observés pour les paramètres de libido et de production entre les 2 groupes de taureaux pendant la période d'adaptation.**

| | **Treact** | **Tejac** | **Vol** | **C°** | **Nbrspz** | **Mob** |
|---|---|---|---|---|---|---|
| **G1** | **89 ± 180** | **323 ± 280** | **6.7 ± 1.4** | **1.44 ± 0.3** | **9.4 ± 2.3** | **63 ± 3.1** |
| **G2** | **320 ± 302** | **780 ± 478** | **7 ± 1.6** | **1.45 ± 0.32** | **10.3 ± 3.4** | **64 ± 3.1** |
| p | **<0.0008** | **<0.0001** | NS | NS | NS | **0.07** |

| | | | | | | |
|---|---|---|---|---|---|---|
| Vol : Volume en mL C° : concentration en milliard de spermatozoïdes/mL Nbrspz : nombre de spermatozoïdes (en milliards) Mob : mobilité NS : Non significatif | | | | | | |

Les observations des deux groupes de taureaux permettent de constater qu'ils sont bien significativement différents pour ce qui concerne la libido.

Ainsi, les temps de réaction et d'éjaculation sont en moyenne de 320 secondes et 780 secondes pour le groupe 2, contre 89 secondes et 323 secondes pour le groupe 1, ce qui est conforme aux attentes.

En revanche, les paramètres de production et notamment les données quantitatives sont comparables entre les deux lots.

### 2- Analyse de l'influence des traitements

### 2-1- Sur l'ensemble des taureaux

L'influence du traitement a été analysée dans un premier temps sur l'ensemble des taureaux indépendamment de leur groupe d'affectation initiale.

L'analyse a porté sur la période des 4 semaines expérimentales. Les paramètres de libido et de production des 60 éjaculats « témoins » et des 60 éjaculats « phéromones » ont été comparés. Les résultats sont présentés dans le tableau 3 ci-dessous, qui montre les paramètres de libido et de production des éjaculats récoltés avec ou sans traitement, tous taureaux confondus.

**Tableau 3 : Comparaison des paramètres de libido et de production des éjaculats récoltés pendant la période expérimentale**

| | **N** | **Treact** | **Tejac** | **Vol** | **C°** | **Nbrspz** | **Mob** |
|---|---|---|---|---|---|---|---|
| **Te** | **60** | **310±383** | **707±637** | **7 ±1.4** | **1.47 ±0.2** | **10.2 ±2.2** | **63. ±4** |
| **Ph+** | **60** | **232± 342** | **615 ±518** | **7.2± 1.4** | **1.56 ±0.22** | **11.1± 2.4** | **64± 4** |
| Δ | | **-77sec -25%** | **- 91 secs -13%** | **+0.15 +2%** | **+0.09 +6%** | **+0.9 9%** | |
| p | | **=0,08** | **=0,06** | **NS** | **=0,008** | **0,02** | |

| | | | | | | | |
|---|---|---|---|---|---|---|---|
| N : nombre d'éjaculats Δ : différence entre les 2 variables p : probabilité sur 1 que l'événement observé soit dû au hasard | | | | | | | |

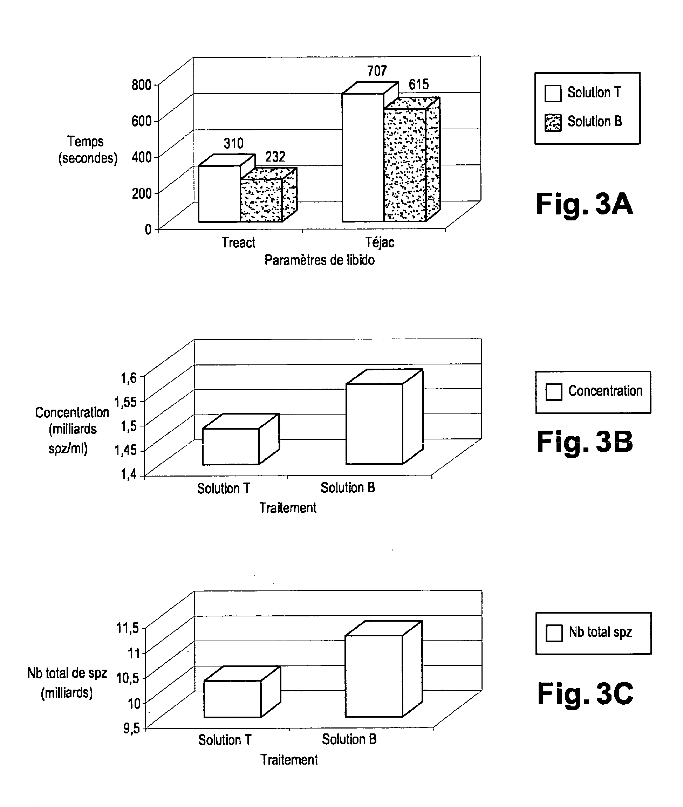

Sur l'ensemble des 10 taureaux de l'essai, l'utilisation des molécules se traduit par une réduction des temps de réaction (Treact) et d'éjaculation (Tejac) de respectivement 25% et 13% (figure 3A).

Parallèlement la concentration (C°) et le nombre total de spermatozoïdes récoltés par éjaculat (Nbrspz) sont améliorés de 6% (p=0,008) et 9% (p=0,02) respectivement (figures 3B et 3C) tandis que le volume ne varie pas de façon significative (+2%).

Pour tous les paramètres analysés, l'effet individuel « taureau » a été significatif. L'effet individuel « taureau » s'entend du comportement propre à chaque animal et susceptible d'influencer les paramètres mesurés.

Aucune interaction « taureau*traitement » n'a été mise en évidence. Tous les taureaux présentent une amélioration plus ou moins marquée grâce au traitement, ladite amélioration étant due au seul traitement.

### 2-2- Sur les taureaux à libido « normale » (G1)

Afin de comparer l'effet des molécules sur les taureaux à libido normale, une analyse statistique (Proc GLM, SAS) a été réalisée au sein du groupe pendant la période expérimentale.

Les facteurs de variation retenus ont été l'effet individuel « taureau », le traitement et leur interaction 2 à 2.

Les résultats sont présentés dans le tableau 4 ci-dessous.

**Tableau 4 : Comparaison des paramètres de libido et de production des éjaculats récoltés pendant la période expérimentale sur les taureaux du groupe 1**

| | **N** | **Treact** | **Tejac** | **Vol** | **C°** | **Nbrspz** | **Mob** |
|---|---|---|---|---|---|---|---|
| **Solution T** | **30** | **45± 36** | **271 ±140** | **7.07 ±1.3** | **1.42± 0.21** | **10± 2.2** | **63 ±3.6** |
| **Solution B** | **30** | **38 ±32** | **255± 64** | **7.13 ±1.1** | **1.56± 0.23** | **11.1± 2.3** | **64 ±3.8** |
| p | | **P<** | **NS** | **NS** | **P<0,004** | **P=0,07** | |

On constate que l'effet « taureau » joue significativement sur le temps de réaction mais n'influence pas le temps d'éjaculation. Il ressort également pour la concentration.

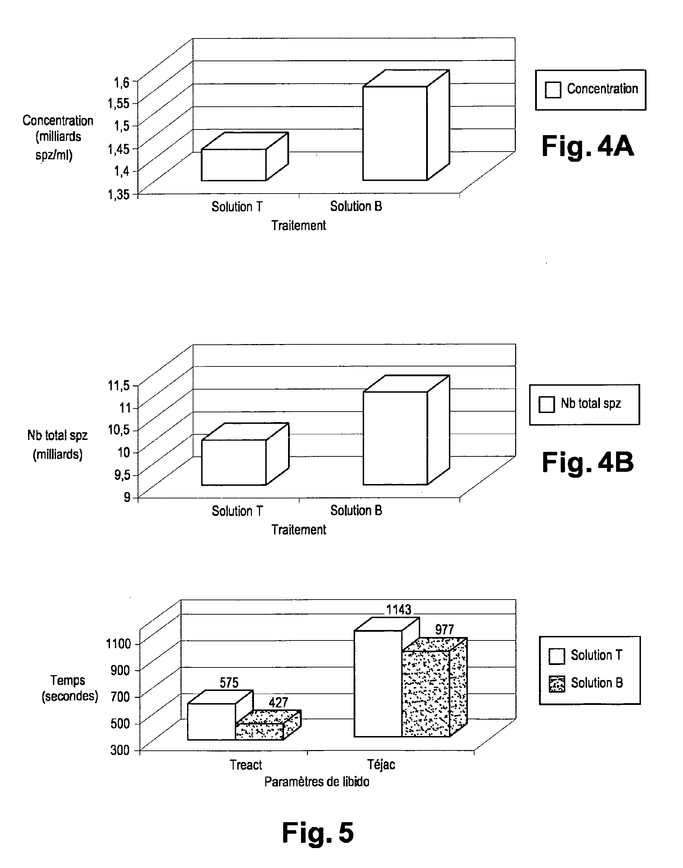

L'emploi des phéromones réduit le temps de réaction de 15%, et permet un accroissement de la concentration des éjaculats (figure 4A) de 10% (p<0,004) et du nombre total de spermatozoïdes (figure 4B) de 11% (p=0,07).

### 2-2- Sur les taureaux à « faible » libido (G2)

Le modèle appliqué au groupe G1 l'a ensuite été à l'autre lot de taureaux G2. Les résultats globaux sont présentés dans le tableau 5 ci-dessous.

**Tableau 5 : Comparaison des paramètres de libido et de production des éjaculats récoltés pendant la période expérimentale sur les taureaux du groupe 2**

| | **N** | **Treact** | **Tejac** | **Vol** | **C°** | **Nbrspz** | **Mob** |
|---|---|---|---|---|---|---|---|
| **Solution T** | **30** | **575± 389** | **1143± 642** | **7± 1.4** | **1.52 ±0.27** | **10.4± 2.3** | **63.5 ±4.3** |
| **Solution B** | **30** | **427± 398** | **977± 522** | **7.2 ±1.6** | **1.55± 0.21** | **11.1 ±2.5** | **64 ±4.1** |
| Δ | | **-148 sec -26%** | **-165 sec -14%** | **0.23 +3%** | **+0.03 +3%** | **+0.67 +6%** | |
| p | | **P=0,1** | **P=0,07** | **NS** | **NS** | **NS** | **NS** |

L'effet individuel « taureau » apparaît significatif pour tous les paramètres.

Le traitement, lui, n'influence que les paramètres de libido (figure 5) : gain de 26% pour Treact (p=0,1) et de 14% pour Tejac (p=0,07).

L'interaction « taureau*traitement » tend à être significative (p=0,09) pour la concentration. Cette observation tient au fait qu'un taureau sur les 5 du groupe voit sa concentration en spermatozoïdes baisser de façon nette du fait du traitement (1,76 milliards de spz/ml en témoin versus 1,55milliards/ml avec les phéromones) alors que pour les autres individus le traitement induit une augmentation de cette donnée mais dans des proportions moindres.

Les autres paramètres ne semblent pas influencés.

### 2-4- Sur l'effet individuel « taureau »

Les effets « taureau » ont été analysés pour chaque individu et chaque paramètre en fonction du traitement et sont présentés dans les tableaux 6 et 7 ci-dessous.

**Tableau 6 : Effet du traitement pour chaque taureau du groupe 1 sur les paramètres de libido et de production**

| Animal | Traitement | Treact | Tejac | Vol | C° | Nbrspz | Mob |
|---|---|---|---|---|---|---|---|
| Vagan | Solution T | 53 | 349 | 7 | 1.6 | 11.2 | 64 |
| | Solution B | 59 | 323 | 6.3 | 1.8 (a) | 11.8 | 65 |
| Valier | Solution T | 59 | 262 | 7.6 | 1.4 | 10.7 | 63.3 |
| | Solution B | 71.2 | 262 | 7.4 | 1.5 | 11 | 64.2 |
| Valrus | Solution T | 25.3 | 239 | 6.9 | 1.4 | 9.8 | 63 |
| | Solution B | 20 | 255 | 7.5 | 1.5 | 11.2 | 65 |
| Vathi | Solution T | 58 | 281 | 6.2 | 1.5 | 9.2 | 62 |
| | Solution B | 21 (b) | 209 (c) | 7.3 d | 1.6 (e) | 12 (f) | 62 |
| Volcain | Solution T | 29 | 224 | 7.7 | 1.2 | 9.1 | 62.5 |
| | Solution B | 18 | 223 | 7.1 | 1.3 | 9.4 | 63 |

| | | | | | | | |
|---|---|---|---|---|---|---|---|
| a :p=0,07 ; b : p=0,06 ; c : p=0,03 ; d : p=0,005 ; e : p=0,05 ; f : p=0,003 | | | | | | | |

Dans le groupe 1, on observe que 2 taureaux sur 5 voient leur temps de réaction augmenter sous l'effet du traitement. Toutefois, sous l'effet du traitement, le temps de réaction cumulé pour les 5 animaux est diminué par rapport au témoin (189,2 secondes avec traitement contre 224,3 secondes sans traitement, soit un gain de temps de 15,6%).

Le temps d'éjaculation est amélioré pour 2 animaux dont un de façon significative (p=0,03), il ne change pas pour 2 autres et tend à augmenter pour le dernier. En cumul, le traitement permet de diminuer de 6,1% le temps d'éjaculation des 5 taureaux confondus (1356 secondes sans traitement, contre 1272 secondes avec traitement).

**Tableau 7 : Effet du traitement pour chaque taureau du groupe 2 sur les paramètres de libido et de production**

| Animal | Traitement | Treact | Tejac | Vol | C° | Nbrspz | Mob |
|---|---|---|---|---|---|---|---|
| vador | Solution T | 581 | 1144 | 8 | 1.6 | 12.4 | 65 |
| | Solution B | 564 | 1032 | 7.6 | 1.7 | 13.7 | 67 |
| vanzi | Solution T | 316 | 709 | 6 | 1.4 | 9.6 | 62.5 |
| | Solution B | 321 | 817 | 7.2 | 1.5 | 11 | 62.5 |
| Virtel | Solution T | 1099 | 2237 | 5 | 1.7 | 8.8 | 61 |
| | Solution B | 633 | 1630 | 5.1 | 1.6 | 7.7 | 60 |
| Voladi | Solution T | 472 | 910 | 7 | 1.7 | 11.7 | 65 |
| | Solution B | 392 | 834 | 7.2 | 1.7 | 12 | 67 |
| Vosac | Solution T | 405 | 713 | 8.4 | 1.14 | 9.6 | 64 |
| | Solution B | 225 | 571 | 8.5 | 1.29 | 11 | 63 |

Dans le groupe 2, le traitement permet un gain cumulé de 25,7% sur le temps de réaction, et de 14,5% sur le temps d'éjaculation, soit respectivement 738 et 829 secondes pour les 5 taureaux.

Cela signifie que le traitement permet de gagner 13 minutes 48 secondes sur le temps total passé en salle de monte lorsque les 5 taureaux font partie de la même session de monte.

### EXEMPLE II

### I- MATERIEL ET METHODES

### 1- Matériel animal

Vingt jeunes taureaux de race montbéliarde, âgés de 15 à 18 mois et faisant partie d'un lot d'animaux en contrôle de fonction sexuelle (CFS) ont été inclus dans la seconde étude. Ces taureaux, recrutés en fermes à l'âge de 2 à 3 mois, ont été élevés ensuite dans des conditions identiques et sans contact avec des femelles.

En l'absence de données préalables de libido, ils ont été répartis de façon aléatoire en deux lots J et K de 10 individus.

### 2- Solution utilisée

La solution A+B a été utilisée et conditionnée sous forme de spray expérimental, dans des pulvérisateurs.

Une version témoin (solution T dans le tableau 8) a été réalisée, en utilisant uniquement le solvant employé également pour la mise en solution des molécules actives de la solution A+B.

Les pulvérisateurs portaient des numéros de code (1, 2) dont le sens était inconnu des taureliers, sauf de la personne chargée d'administrer le traitement en début de collecte.

### 3- Organisation générale des essais : distribution des solutions

Le tableau 8 ci-dessous reprend, pour chacun des groupes de taureaux, les expérimentations réalisées.

L'essai a duré 5 semaines, dont une d'adaptation pendant laquelle tous les animaux ont reçu la solution témoin et n'ont été prélevés qu'une seule fois. La solution a été conditionnée sous forme de spray, comme dans l'exemple I précédent.

L'administration des solutions a eu lieu systématiquement en début de collecte, sur l'ensemble des taureaux sous forme d'une double pulvérisation du mélange par naseau.

Les molécules utilisées étant très volatiles, le lot « témoin » a toujours commencé la récolte, afin de ne pas saturer l'atmosphère de la salle de collecte en molécules actives. Tous les animaux ont reçu le traitement à leur arrivée en salle de monte, qui s'est fait de façon aléatoire au sein de chaque groupe.

**Tableau 8 : Organisation générale des essais pour les taureaux adultes**

| | Semaine 1 | Semaine 2 | Semaine 3 | Semaine 4 | Semaine 5 |
|---|---|---|---|---|---|
| Groupe J | Solution T | Solution T | Solution A+B | Solution T | Solution A+B |
| | Adaptation | Expérimentation | Expérimentation | Expérimentation | Expérimentation |
| Groupe K | Solution T | Solution A+B | Solution T | Solution A+B | Solution T |
| | Adaptation | Expérimentation | Expérimentation | Expérimentation | Expérimentation |

### 4- Enregistrement des paramètres comportementaux et de production

Pendant toute la période expérimentale de l'essai, soit 4 semaines, tous les animaux ont été soumis à un rythme de 2 collectes hebdomadaires d'un éjaculat (2*1).

La préparation des taureaux et du boute-en-train, ainsi que le mode d'administration de la solution et de récupération des éjaculats, ont été les mêmes que pour l'exemple I.

Les observations et l'enregistrement des paramètres de libido et de production sont les mêmes que pour l'exemple I.

### II- RESULTATS

### 1- Analyse des effets du traitement, du groupe, du taureau intra-groupe et de leurs interactions

Les valeurs moyennes obtenues pour chaque paramètre mesuré sur l'ensemble des 20 taureaux confondus et en fonction du traitement sont présentées dans le tableau 9 ci-dessous. Elles concernent les 4 semaines de la période expérimentale.

**Tableau 9 : valeurs moyennes des paramètres mesurés en fonction du traitement, tous taureaux confondus**

| **Traitement** | **effectifs** | **Treact** | **Tejac** | **Vol** | **C°** | **Nbspz** | **Mob** |
|---|---|---|---|---|---|---|---|
| Solution T | 80 | 33,5 | | 3,36 | 1,01 | 3,39 | 56,34 |
| Solution A+B | 80 | 31,1 | 297 | 3,23 | 1,09 | 3,45 | 56,02 |
| p | | NS | P<0,08 | NS | P=0,11 | NS | NS |

Sur l'ensemble des 20 jeunes taureaux de l'essai, il apparaît que l'utilisation du cocktail de phéromones tend à abaisser le temps d'éjaculation (347 secondes pour les témoins, contre 297 secondes pour les traités, soit un gain de temps de 14%) et à augmenter la concentration moyenne de l'éjaculat (1,01 milliards/ml pour le groupe témoin contre 1,09 milliards/ml pour le groupe traité soit un gain de 9% (p<0,11)).

Les autres paramètres ne semblent pas modifiés par le traitement.

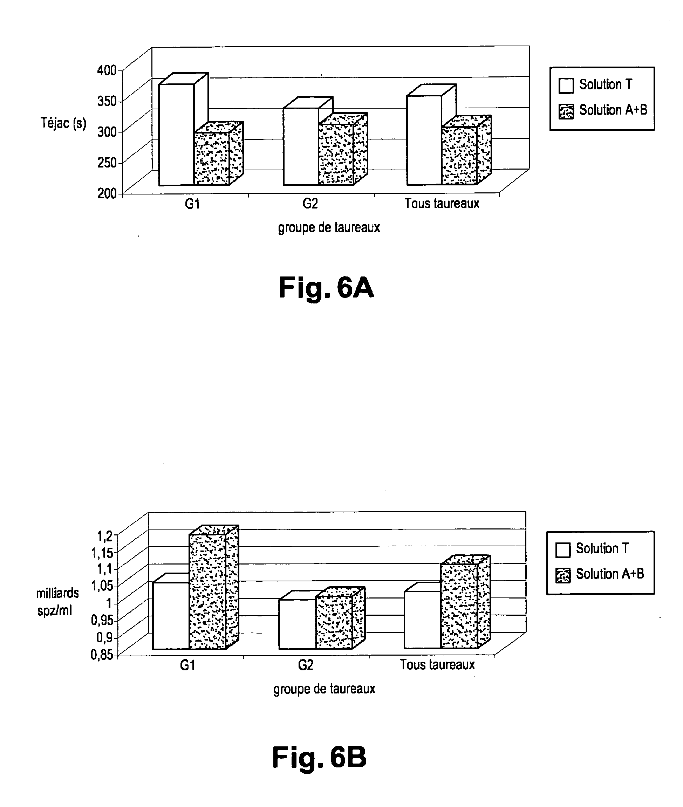

L'influence du traitement sur le temps d'éjaculation et la concentration, par groupe de taureaux et globalement, est représentée sur les figures 6A et 6B.

Ainsi, on observe que pour tous les paramètres mesurés, l'effet individuel « taureau » ressort très significatif (p<0,0001). Par contre aucune interaction « taureau*traitement » n'est observée.

Il apparaît que le traitement est d'autant plus efficace que la libido initiale des taureaux est mauvaise, et que le temps d'éjaculation témoin est élevé.

Sur les 20 taureaux de l'essai, seul un (le T15) a un comportement atypique et voit son temps d'éjaculation augmenter avec le traitement.

De même, pour les individus ayant un temps d'éjaculation témoin inférieur à 200 secondes, correspondant à une bonne libido initiale, le traitement ne semble pas apporter d'amélioration significative sur la libido.

### EXEMPLE III

### I- MATERIEL ET METHODES

### 1- Matériel animal

Un lot de dix taureaux adultes, âgés de 4 ans à 5,5 ans, de races Prim Holstein ou Normande, a été inclus dans la première étude. Ces animaux ont été recrutés dans la filière « insémination animale » dès l'âge de 2 à 3 mois et depuis n'ont jamais été en contact avec des femelles bovines. Ils ont toujours été élevés en boxes individuels (période de pré-testage ou période de production) ou en lots de 4 à 6 animaux de même âge et de même sexe (période de lay-off). Les conditions d'alimentation et d'hébergement ont toujours été identiques pour les animaux de l'essai, avant et pendant l'essai.

Comme pour l'exemple I, les taureaux ont été divisés en deux lots en fonction de leur libido : Groupe N « libido normale » et Groupe L « faible libido ».

### 2- Solutions utilisées

La solution A a été utilisée et conditionnée sous forme de spray expérimental, dans des pulvérisateurs.

Une version témoin (solution T dans le tableau 10) a été réalisée, en utilisant uniquement l'excipient employé également pour la mise en solution des molécules actives de la solution A.

Les pulvérisateurs portaient des numéros de code (1, 2) dont le sens était inconnu des taureliers, sauf de la personne chargée d'administrer le traitement en début de collecte.

### 3- Organisation générale des essais : distribution des solutions

Le tableau 10 ci-dessous reprend pour chacun des groupes de taureaux les expérimentations réalisées.

L'essai a duré au total 6 semaines.

Les semaines se sont organisées comme pour l'exemple 1.

**Tableau 10 : Organisation générale des essais pour les taureaux adultes**

| | Semaine 1 | Semaine 2 | Semaine 3 | Semaine 4 | Semaine 5 | Semaine 6 |
|---|---|---|---|---|---|---|
| Groupe N | Solution T | Solution T | Solution T | Solution A | Solution T | Solution A |
| Libido normale | Adaptation | Adaptation | Expérimentation | Expérimentation | Expérimentation | Expérimentation |
| Groupe L | Solution T | Solution T | Solution A | Solution T | Solution A | Solution T |
| Faible libido | Adaptation | Adaptation | Expérimentation | Expérimentation | Expérimentation | Expérimentation |

La solution a été conditionnée sous forme de spray, comme dans l'exemple I précédent.

L'administration des solutions a eu lieu systématiquement en début de collecte sur l'ensemble des taureaux sous forme d'une double pulvérisation du mélange par naseau.

Les molécules utilisées étant très volatiles, le lot « témoin » a toujours commencé la récolte, afin de ne pas saturer l'atmosphère de la salle de collecte en molécules actives. Tous les animaux ont reçu le traitement à leur arrivée en salle de monte, qui s'est fait de façon aléatoire au sein de chaque groupe.

### 4- Enregistrement des paramètres comportementaux

Le rythme des collectes, la préparation des taureaux et l'enregistrement des paramètres comportementaux ont été réalisés dans des conditions identiques à celles de l'exemple I.

### 5- Évaluation des paramètres biologiques

La collecte de la semence a été réalisée dans des conditions identiques à celles décrites pour l'exemple I.

Les paramètres biologiques quantitatifs et qualitatifs ont été enregistrés comme dans l'exemple I.

### II- RESULTATS

### 1- Comparaison initiale des taureaux

### 1-1 Comparaison entre les groupes de taureaux

Les performances des deux lots de taureaux (Groupe N et Groupe L) ont été comparées à partir de l'ensemble des paramètres enregistrés pendant la période d'adaptation.

L'observation a porté sur 5 collectes de 2 éjaculats (rang 1 et rang 2), soit 42 éjaculats pour le groupe N et 47 éjaculats pour le groupe L, en raison de refus de sauts et de données manquantes. Les résultats sont présentés dans le tableau 11 ci-dessous, et correspondent à l'ensemble des éjaculats prélevés quel que soit le rang de collecte:

**Tableau 11 : comparaison des moyennes et écarts-types observés pour les paramètres de libido et de production entre les 2 groupes de taureaux pendant la période d'adaptation.**

| | **Treact** | **Tejac** | **Vol** | **C°** | **Nbrspz** | **Mob** |
|---|---|---|---|---|---|---|
| **Groupe N** | **139 ± 357** | **378 ± 361** | **5.2 ± 1.6** | **1.21 ± 0.48** | **6.3 ± 3.4** | **84.5 ± 3.3** |
| **Groupe L** | **895± 1041** | **1368 ± 1176** | **6,4 ± 3** | **1.37 ± 0.5** | **8.9 ± 5.3** | **84.5 ± 3.6** |
| p | **<0.0001** | **<0.0001** | **0.03** | **NS** | **0.012** | **NS** |

On observe que la répartition des taureaux dans les 2 groupes est cohérente car les animaux du groupe L sont significativement plus longs à s'exciter (Treact= 895 secondes pour le groupe L, contre 139 secondes seulement pour le groupe N) et à donner leur sperme (Tejac= 1368 secondes pour le groupe L, et 378 secondes pour le groupe N).

On observe également que les paramètres quantitatifs du spermogramme sont significativement supérieurs chez les taureaux du groupe L (p=0.03 pour le volume, p=0.012 pour le nombre de spermatozoïdes totaux et une concentration de 1,37 milliards/ml pour le groupe L, contre 1,21 milliards/ml pour le groupe N).

Du fait de ces différences entre groupes, la suite de l'analyse statistique s'est faite séparément entre les 2 lots de taureaux, excepté pour les paramètres qualitatifs qui ne différent pas.

### 2-2 Comparaison entre les rangs d'éjaculat des taureaux

Les analyses ont ensuite été faites en intégrant le facteur « rang d'éjaculat ».

### 2-2-1- Comparaison entre les éjaculats de rang 1 des taureaux des deux groupes

Les résultats obtenus pour chaque groupe de taureaux sont présentés dans le tableau 12 ci-dessous.

**Tableau 12 : comparaison des paramètres de libido et de production entre les 2 groupes de taureaux pour les éjaculats de rang 1**

| | Treact | Tejac | Vol | C° | Nbspz | Mob |
|---|---|---|---|---|---|---|
| Groupe L N=22 | 1248 | 1724 | 7,1 | 1,65 | 11,65 | 85 |
| Groupe N N=23 | 68,3 | 339,4 | 5,25 | 1,37 | 7,24 | 85 |
| p | 0,0001 | 0,0001 | 0,02 | 0,05 | 0,004 | NS |

Les différences observées entre les 2 groupes sur l'ensemble des éjaculats récoltés se retrouvent de façon plus marquée pour les éjaculats de rang 1.

Les paramètres de libido sont très significativement différents puisqu'on passe d'un temps de réaction moyen de 1 minute 8 secondes pour le groupe N, à 20 minutes et 48 secondes pour les animaux « lents », et d'un temps d'éjaculation moyen de 5 minutes 39 secondes pour les taureaux à libido « normale », à 28 minutes et 44 secondes en moyenne pour ceux du groupe L.

Les paramètres quantitatifs du spermogramme sont également significativement différents entre les groupes, en faveur du groupe L, tandis que les paramètres qualitatifs (% mobiles) sont identiques.

### 2-2-2- Comparaison entre les éjaculats de rang 2 des taureaux des deux groupes

Les résultats obtenus pour chaque groupe de taureaux sont présentés dans le tableau 13 ci-dessous.

**Tableau 13 : comparaison des paramètres de libido et de production entre les 2 groupes de taureaux pour les éjaculats de rang 2**

| | Treact | Tejac | Vol | C° | Nbspz | Mob |
|---|---|---|---|---|---|---|
| Groupe L N=22 | 508 | 976 | 5,64 | 1,06 | 5,92 | 82,2 |
| Groupe N N=23 | 207 | 415 | 5,24 | 1,05 | 5,47 | 82,3 |
| p | 0,1 | 0,02 | NS | NS | NS | NS |

Pour les éjaculats de rang 2, les différences entre groupes s'estompent et seuls les paramètres de libido tendent à être significativement différents (Treact, p<0,1 et Tejac, p<0,02). Ainsi, le temps d'éjaculation moyen de 6 minutes 55 secondes dans le groupe N passe à 16 minutes et 16 secondes dans le groupe L. Il est intéressant d'observer que dans le groupe L, les paramètres de libido sont améliorés lors du 2^{ème} saut tandis qu'ils sont détériorés dans le groupe N.

Les paramètres quantitatifs et qualitatifs du spermogramme sont très proches entre les 2 groupes pour ce qui concerne le 2^{ème} éjaculat.

### 3- Influence des traitements

### 3-1- Effets du traitement en fonction du groupe sur les éjaculats de rang 1

### 3-1-1- Groupe N (taureaux à libido normale)

Dans ce groupe, le traitement a un effet positif sur les paramètres de production tandis que les paramètres de libido ne sont pas significativement modifiés.

L'effet « taureau » ne ressort que pour les caractères quantitatifs du spermogramme et aucune interaction « taureau*traitement » (Toro*trait dans le tableau 14) n'apparaît.

Ces résultats sont présentés dans le tableau 14.

**Tableau 14 : Moyennes des paramètres de libido et de production et analyse de facteurs de variation pendant la période expérimentale. Groupe N. (n=30 observations par traitement)**

| | Treact | Tejac | Vol | C° | Nbspz | Mob |
|---|---|---|---|---|---|---|
| Taureau | NS | NS | P<0,0001 | P<0,0001 | P<0,0001 | NS |
| Traitement | NS | NS | NS | P<0,05 | P<0,05 | P<0,02 |
| Toro*trait | NS | NS | NS | NS | NS | NS |
| Solution T | 84,7 | 316 | 5,03 | 1,35 | 6,73 | 84,5 |
| Solution A | 94,4 | 340 | 5,15 | 1,50 | 7,75 | 86,3 |
| Δ(A -T) | +9,7s | +24s | 0,12 | 0,15 | +1,02 | +1,8 |

### 3-1-2- Groupe L (taureaux à faible libido)

Dans ce groupe (n=30 observations par traitement), les effets individuels « taureau » ressortent de façon marquée, quel que soit le paramètre analysé, tandis que le traitement est significatif pour le pourcentage de spermatozoïdes mobiles, pour le volume de l'éjaculat et le nombre total de spermatozoïdes récoltés (p<0,07).

Ainsi, le traitement réduit le temps d'éjaculation par taureau de 393 secondes soit 6 minutes 33 secondes. Ces données sont résumées dans le Tableau 15.

**Tableau 15 : Moyennes des paramètres de libido et de production et analyse de facteurs de variation pendant la période expérimentale.**

| | Treact | Tejac | Vol | C° | Nbrspz | Mob |
|---|---|---|---|---|---|---|
| Taureau | P<0,0001 | P<0,0001 | P<0,0001 | P<0,0001 | P<0,0001 | P<0,07 |
| Traitement | NS | NS | 0,06 | NS | P<0,07 | P<0,05 |
| Toro*trait | NS | P<0,07 | NS | NS | NS | NS |
| Solution T | 1078 | 2179 | 6,6 | 1,51 | 9,89 | 86,2 |
| Solution A | 846 | 1786 | 5,88 | 1,47 | 8,18 | 84,3 |
| Δ(A-T) | -232s | -393s | -0,72 | -ô,04 | -1,71 | -1,9 |

### 3-2- Effets du traitement en fonction du groupe sur les éjaculats de rang 2

### 3-2-1- Groupe N (taureaux à libido normale)

Chez les taureaux à libido « normale », (n=30 observations par traitement), les molécules utilisées tendent à diminuer le temps d'éjaculation (p<0,07), mais également la concentration et le nombre de spermatozoïdes (p<0,01).

Elles ont un effet positif (p<0,02) sur le pourcentage de spermatozoïdes motiles.

L'influence du taureau ne se fait ressentir que sur les paramètres quantitatifs du spermogramme tandis qu'aucune interaction « taureau*traitement » ne ressort (tableau 16).

**Tableau 16 : Moyennes des paramètres de libido et de production et analyse de facteurs de variation pendant la période expérimentale, 2^{ème} éjaculat.**

| | Treact | Tejac | Vol | C° | Nbspz | Mob |
|---|---|---|---|---|---|---|
| Taureau | NS | NS | P<0,0001 | P<0,0006 | P<0.0001 | NS |
| Traitement | NS | P<0,07 | NS | P<0,01 | P<0,01 | P<0,02 |
| Toro*trait | NS | NS | NS | NS | NS | NS |
| Solution T | 180 | 415 | 4,65 | 1,22 | 5,76 | 82,2 |
| Sol ution A | 71 | 258 | 4,31 | 0,99 | 4,25 | 84,5 |
| Δ(A-T) | -109s | -157s | -0,34 | -0,23 | -1,51 | +2,3 |

### 3-2-2- Groupe L (taureaux à faible libido)

Dans ce lot d'animaux (n=30 observations par traitement), seul l'effet individuel « taureau » ressort sur l'ensemble des paramètres étudiés, excepté pour le pourcentage de spermatozoïdes motiles qui est le seul influencé par le traitement. Paradoxalement le traitement tend à augmenter les paramètres de libido mais de façon non significative. Ces données sont résumées dans le tableau 17.

**Tableau 17 : Moyennes des paramètres de libido et de production et analyse de facteurs de variation pendant la période expérimentale, 2^{ème} éjaculat.**

| | Treact | Tejac | Vol | C° | Nbspz | Mob |
|---|---|---|---|---|---|---|
| Taureau | P<0,01 | P<0,0001 | P<0,0001 | P<0,01 | P<0,0001 | NS |
| Traitement | NS | NS | NS | NS | NS | P<0,01 |
| Toro*trait | NS | NS | NS | NS | NS | NS |
| Solution T | 312 | 868 | 5,74 | 0,91 | 5,07 | 84,6 |
| Solution A | 494 | 1137 | 5,28 | 0,88 | 4,84 | 81,9 |
| Δ(A-T) | +182 | +269 | -0,46 | -0,03 | -0,23 | -2,7 |

### EXEMPLE IV

### I- MATERIEL ET METHODES

### 1- Matériel animal

Des lots de dix taureaux adultes de races différentes ont été testés sur plusieurs sites d'expérimentation. Chacun de ces lots a été divisé en deux catégories : animaux témoins et animaux traités. Dans chaque site, les taureaux ont été répartis en tenant compte des données d'âge et de race de façon équilibrée entre les deux lots, afin que seules les différences de libido soient discriminantes.

Comme pour l'Exemple III, les taureaux ont été divisés en fonction de leur libido : Groupe N, « libido normale », Groupe L, « faible libido ».

Pour chaque site, les races traitées et les rythmes de collecte sont indiqués dans le Tableau 18.

**Tableau 18 : Races et rythmes de collecte dans chaque site**

| Site | Effectifs | Races | Rythmes de collecte |
|---|---|---|---|
| 1 | 5 témoins | Holstein / Normande | 3*1 |
| | 5 traités | | |
| 2 | 5 témoins | Holstein / Normande | 3*2 |
| | 5 traités | | |
| 3 | 5 témoins | Limousine | 2*1 |
| | 5 traités | | |
| 4 | 5 témoins | Holstein | 2*1 |
| | 5 traités | BI d'Aquitaine Simmental | |
| 5 | 5 témoins | Holstein | 2*1 |
| | 5 traités | | |

| | | | |
|---|---|---|---|
| où x*y exprime un nombre x de collectes hebdomadaires de y éjaculats successifs. | | | |

### 2- Solutions utilisées

Une solution témoin (solution T dans le Tableau 19) comprenant du glycérol et de l'eau dans des proportions respectives de 50 / 50 en volume a été utilisée.

Les autres solutions comportant la ou les molécules selon l'invention ont été diluées dans cette solution témoin.

Différentes concentrations en molécule active ou mélange de molécules actives ont été testées.

Les compositions testées sont décrites dans le Tableau 19.

**Tableau 19 : Compositions testées**

| Site | Composition | Code |
|---|---|---|
| Tous les sites | Solution témoin (glycérol:eau, 50:50) | T |
| 1 | 1,2-dichloroéthylène 2,5 pg/mL + squalène 2,5 pg/mL dans la solution témoin | C1 |
| 2 | 1,2-dichloroéthylène 250 pg/mL + squalène 250 pg/mL dans la solution témoin | C2 |
| 3 | 1,2-dichloroéthylène 25 pg/mL + squalène 25 pg/mL + 2-butanone 25 pg/mL dans la solution témoin | C3 |
| 4 | Acide 9-octadécénoïque 25 pg/mL dans la solution témoin | C4 |
| 5 | 2-butanone 25 pg/mL dans la solution témoin | C5 |

Toutes ces compositions ont été conditionnées sous forme de spray dans des pulvérisateurs en aluminium doublé d'un revêtement neutre pour éviter le relarguage de substances parasites.

Sur chaque site, l'équipe de taurellerie disposait de deux pulvérisateurs codés. L'un contenait uniquement la solution témoin, et l'autre la composition contenant une ou un mélange de molécules selon l'invention. Seul le responsable d'équipe pouvait différencier les deux pulvérisateurs, mais n'en connaissait pas le contenu.

### 3- Organisation générale des essais : distribution des solutions

Le Tableau 20 ci-après reprend, pour chacun des groupes de taureaux N et L, les expérimentations réalisées.

L'essai a duré au total 6 semaines. Les deux premières semaines ont été consacrées à l'entraînement des taureaux au rythme de collecte, à la stabilisation des réserves spermatiques et à l'estimation du potentiel de production des individus. Elles ont permis de s'assurer de la pertinence de la répartition entre les deux groupes : différence significative de libido estimée par les temps de réaction et d'éjaculation et absence de différence entre paramètres de production. Les 4 semaines suivantes ont constitué la phase expérimentale proprement dite et ont été programmées en carré latin : chaque lot a subi alternativement la distribution du spray témoin et du traitement et chaque taureau a également été son propre témoin.

**Tableau 20 : Organisation générale des essais par semaine**

| | Adaptation | | Expérimentation | | | |
|---|---|---|---|---|---|---|
| | Sem.1 | Sem. 2 | Sem. 3 | Sem. 4 | Sem. 5 | Sem. 6 |
| Groupe N | Solution T | Solution T | Solution T | Comp. Cx | Solution T | Comp. Cx |
| Groupe L | Solution T | Solution T | Comp. Cx | Solution T | Comp. Cx | Solution T |

| | | | | | | |
|---|---|---|---|---|---|---|
| où Comp. Cx désigne l'une des compositions C1 à C5, selon le site d'expérimentation | | | | | | |

Afin de ne pas saturer l'atmosphère de la salle de monte en molécule active et de ne pas risquer de fausser les résultats, la solution témoin a été systématiquement utilisée avant les compositions Cx.

### 4- Enregistrement des paramètres comportementaux

Le rythme des collectes a été réalisé comme indiqué dans le Tableau 18 ci-dessus. L'enregistrement des paramètres comportementaux a été réalisé dans des conditions similaires à celles de l'Exemple I.

Pour le site 2, les paramètres liés au premier et au deuxième éjaculat ont été pris en compte.

### 5- Analyse statistique

Une analyse statistique a été réalisée conformément à la méthode présentée pour l'Exemple I ci-dessus, pour les paramètres de la libido.

### II- RESULTATS

### 1- Effets du traitement sur les paramètres de la libido

### 1-1- Effets sur le temps de réaction (Treact)

Les effets du traitement pour chaque site et sur l'ensemble des 10 taureaux testés sont présentés dans le Tableau 21.

**Tableau 21 : Effets de chaque composition testée sur le temps de réaction (exprimé en secondes)**

| | Témoins | | | | Traités | | | | | |
|---|---|---|---|---|---|---|---|---|---|---|
| Comp. | **Moly.** | Ecart-type | Maxi | Mini | **Moy.** | Ecart-type | Maxi | Mini | Diff. moy. (%) | p |
| C1 | **261,5** | 533 | 2696 | 6 | **166** | 273,4 | 1360 | 6 | -36 | 0,09 |
| C2 Ejac. 1 | **251** | 842,4 | 5700 | 2 | **460** | 1240,4 | 6600 | 2 | +83 | |
| C2 Ejac. 2 | **76,3** | 125,2 | 720 | 2 | **72,2** | 109,2 | 445 | 2 | -5,4 | NS |
| C3 | **315** | 390,5 | 1498 | 10 | **231** | 334,7 | 1550 | 2 | -27 | 0.13 |
| C4 | **16,7** | 14,7 | 48 | 1 | **11,5** | 11,5 | 45 | 1 | -31 | 0,03 |
| C5 | **807** | 912,8 | 3600 | 4 | **549** | 601 | 2100 | 6 | -33 | 0,002 |

| | | | | | | | | | | |
|---|---|---|---|---|---|---|---|---|---|---|
| où Comp. signifie Composition, Ejac. signifie Ejaculat, Moy. signifie Moyenne, Diff. moy. signifie Différence moyenne, NS signifie Non Significatif | | | | | | | | | | |

Pour l'ensemble des compositions testées, à l'exception de la composition C2, on observe que le traitement permet une réduction sensible du temps de réaction (27 % à 36 % de réduction en moyenne par rapport aux témoins).

Ces résultats montrent que les compositions conformes à l'invention C1, C3, C4 et C5 ne modifient pas les temps de réaction minimaux des taureaux. En revanche, on constate qu'elles induisent une nette diminution des temps de réaction maximaux. En outre, elles tendent à réduire la variabilité entre les éjaculats et à homogénéiser les temps de réaction observés.

Ces résultats démontrent ainsi que même à une concentration aussi faible que 2,5 pg/ml (composition C1), les molécules selon l'invention sont aptes à stimuler efficacement la libido des taureaux.

Pour la composition C2, dans laquelle les molécules sont présentes à une concentration de 250 pg/ml, on observe tout de même une amélioration du temps de réaction pour l'éjaculat de rang 2.

### 1-2- Effets sur le temps d'éjaculation (Tejac)

Les effets du traitement pour chaque site et sur l'ensemble des 10 taureaux testés sont présentés dans le Tableau 22 ci-après.

**Tableau 22 : Effets de chaque composition testée sur le temps d'éjaculation (exprimé en secondes)**

| | Témoins | | | | Traités | | | | | |
|---|---|---|---|---|---|---|---|---|---|---|
| Comp. | **Moy.** | Ecart-type | Maxi | Mini | **Moy.** | Ecart-type | Maxi | Mini | Diff. moy. (%) | p |
| C1 | **678,5** | 772,5 | 3444 | 184 | **535** | 441 | 2385 | 164 | -21 | 0.03 |
| C2 Ejac. 1 | **474,5** | 882,7 | 5820 | 43 | **736** | 1283 | 6660 | 100 | +55 | |
| C2 Ejac. 2 | **427** | 501,4 | 3040 | 30 | **308,6** | 217 | 980 | 48 | -28 | |
| C3 | **1138** | 388 | 2344 | 520 | **985** | 411.5 | 2160 | 562 | -13 | 0,02 |
| C4 | **35,6** | 21,1 | 83 | 5 | **29,2** | 18,9 | 78 | 5 | -18 | 0.06 |
| C5 | **1232,5** | 1308,8 | 3600 | 135 | **935** | 726 | 2877 | 165 | -24 | 0.005 |

| | | | | | | | | | | |
|---|---|---|---|---|---|---|---|---|---|---|
| où Comp. signifie Composition, Ejac. signifie Ejaculat, Moy. signifie Moyenne, Diff. moy. signifie Différence moyenne | | | | | | | | | | |

Les résultats ci-dessus démontrent qu'à l'exception de la composition C2, les compositions conformes à l'invention testées réduisent de façon significative, de 13 à 28 % suivant les compositions, le temps d'éjaculation. Ceci est également vérifié pour la composition C1, dans laquelle la concentration en molécules est aussi basse que 2,5 pg/ml.

Cet effet s'observe également de manière significative pour la composition C2, au niveau de l'éjaculat de rang 2.

Comme pour le temps de réaction, on observe que les compositions conformes à l'invention induisent principalement une diminution des temps d'éjaculation maximaux, ainsi que de la variabilité entre les taureaux testés.

Ces résultats confortent la conclusion selon laquelle les molécules testées, aux concentrations testées, présentent un effet biologique important sur la libido des taureaux.

A une concentration totale en molécules spécifiques de l'oestrus aussi haute que 500 pg/mL (composition C2), l'effet bénéfique sur les temps de réaction et d'éjaculation est également observé au niveau de l'éjaculat de rang 2, récolté le même jour que l'éjaculat de rang 1. Cet effet bénéfique est différé dans le temps par rapport à l'administration de la composition, mais néanmoins significatif.

### 2- Analyse des effets individuels « taureau » et des interactions « taureau - traitement »

Quels que soient les sites et les paramètres considérés, il ressort des résultats obtenus que l'effet individuel « taureau » est toujours apparu très significatif. Aucune interaction « taureau / traitement » n'est apparue comme significative, y compris pour les paramètres de libido, temps de réaction et temps d'éjaculation, lorsque l'effet positif du traitement a été constaté. Ces données ont été constantes quelles que soient les molécules ou leurs mélanges utilisés, ce qui conforte l'idée que les molécules selon l'invention agissent de façon identique sur la quasi-totalité des taureaux, dans des proportions variables cependant en fonction de la libido initiale des animaux.

Afin d'illustrer ces données, les effets « taureaux » observés sur le site 3 sur les paramètres de libido influencés par le traitement, sont présentés ci-après à titre d'exemple, car représentatifs de ce qu'on obtient sur l'ensemble des autres sites.

Le Tableau 23 ci-après présente les résultats obtenus en termes de temps de réaction pour chaque taureau, en fonction de la présence ou de l'absence de traitement. Chaque moyenne est calculée à partir de 4 éjaculats (rythme 2*1) collectés pendant les 4 semaines de période expérimentale.

**Tableau 23 : Illustration de l'effet individuel « taureau » et de l'absence d'interaction « taureau / traitement » sur le temps de réaction (Treact), exprimé en secondes, sur le site 3 (composition C3)**

| | Témoins | | | | Traités | | | | | |
|---|---|---|---|---|---|---|---|---|---|---|
| Treact | **Moly.** | Ecart-type | Mini | Maxi | **Moy.** | Ecart-type | Mini | Maxi | Diff. moy. | Diff. moy. % |
| taureau 1 | **43** | 24 | 20 | 65 | **38,3** | 21,08 | 16 | 60 | -4,7 | -11 |
| taureau 2 | **55** | 31 | 27 | 89 | **44** | 32,6 | 14 | 85 | -11 | -20 |
| taureau 3 | **79** | 21,7 | 57 | 102 | **59,8** | 12,9 | 48 | 77 | -19,2 | -24 |
| taureau 4 | **539** | 187 | 374 | 733 | **319** | 244,8 | 82 | 638 | -220 | -41 |
| taureau 5 | **24,3** | 13,7 | 10 | 42 | **5** | 2,4 | 2 | 8 | -19,3 | -79 |
| taureau 6 | **770** | 584 | 200 | 1463 | **884** | 452 | 598 | 1550 | 114 | 15 |
| taureau 7 | **897** | 501 | 379 | 1498 | **530** | 436 | 145 | 1124 | -367 | -41 |
| taureau 8 | **139** | 48,7 | 74 | 182 | **93** | 50 | 63 | 167 | -46 | -33 |
| taureau 9 | **115,3** | 104 | 47 | 270 | **56** | 77 | 7 | 170 | -59,3 | -51 |
| taureau 10 | **487** | 191 | 219 | 650 | **282** | 142 | 172 | 470 | -205 | -42 |
| Moyenne | **314,86** | | 140,7 | 509,4 | **231,11** | | 114,7 | 434,9 | -83,75 | -27 |

| | | | | | | | | | | |
|---|---|---|---|---|---|---|---|---|---|---|
| où Moy. signifie Moyenne, Diff. moy. signifie Différence moyenne | | | | | | | | | | |

L'analyse des résultats exposés dans le Tableau 23 permet de constater la grande différence de comportement entre les taureaux, et de retrouver le classement entre taureaux du groupe N, à bonne libido (Taureaux 1, 2, 3, 5 et 9), et taureaux du groupe L, à faible libido (Taureaux 4, 6, 7, 8 et 10), même si l'écart entre les animaux 8 et 9 est modeste.

Pour l'ensemble des taureaux, excepté le taureau 6, l'administration de la composition C3 conforme à l'invention réduit le temps de réaction dans des proportions allant de 11% (pour le taureau 1) à 79% (pour le taureau 5). Seul le taureau 6, appartenant au groupe des individus à faible libido, est pénalisé de 15% par le traitement.

Pour ces taureaux, on observe en outre que les temps de réaction minimal et maximal sont réduits par le traitement.

Le Tableau 24 ci-après présente les résultats obtenus en termes de temps d'éjaculation pour chaque taureau, en fonction de la présence ou de l'absence de traitement. Chaque moyenne est calculée à partir de 4 éjaculats (rythme 2*1) collectés pendant les 4 semaines de période expérimentale.

**Tableau 24 : Illustration de l'effet individuel « taureau » et de l'absence d'interaction « taureau / traitement » sur le temps d'éjaculation (Tejac), exprimé en secondes, sur le site 3 (composition C3)**

| | Témoins | | | | Traités | | | | | |
|---|---|---|---|---|---|---|---|---|---|---|
| Tejac | **Moy.** | Ecart-type | Mini | Maxi | **Moy.** | Ecart-type | Mini | Maxi | Diff. moy. | Diff. moy. % |
| taureau 1 | **755** | 179 | 520 | 954 | **857** | 68 | 779 | 936 | 102 | 14 |
| taureau 2 | **1038** | 101 | 928 | 1157 | **750** | 77 | 670 | 820 | -288 | -28 |
| taureau 3 | **927** | 106 | 802 | 1039 | **658** | 120 | 562 | 833 | -269 | -29 |
| taureau 4 | **1600** | 486 | 1066 | 2030 | **1068** | 255 | 780 | 1397 | -532 | -33 |
| taureau 5 | **1011** | 98 | 880 | 1114 | **636** | 58 | 565 | 685 | -375 | -37 |
| taureau 6 | **1340** | 603 | 641 | 2010 | **1742** | 290 | 1510 | 2160 | 402 | 30 |
| taureau 7 | **1515** | 583 | 1070 | 2344 | **1556** | 260 | 1222 | 1824 | 41 | 3 |
| taureau 8 | **1054** | 126 | 890 | 1192 | **736** | 155 | 600 | 957 | -318 | -30 |
| taureau 9 | **964** | 177 | 800 | 1155 | **672** | 57 | 619 | 736 | -292 | -30 |
| taureau 10 | **1180** | 182 | 950 | 1413 | **1170** | 241 | 863 | 1432 | -10 | -1 |
| Moyenne | **1138,4** | | 854,7 | 1440,8 | 984,5 | | 817 | 1178 | -153,9 | -14 |

| | | | | | | | | | | |
|---|---|---|---|---|---|---|---|---|---|---|
| où Moy. signifie Moyenne, Diff. moy. signifie Différence moyenne | | | | | | | | | | |

Sur l'ensemble des 10 taureaux testés, le gain moyen induit par le traitement par la composition C3 conforme à l'invention est de 154 secondes par taureau, soit 14% du temps d'éjaculation moyen des témoins.

Six taureaux (taureaux 2, 3, 4, 5, 8 et 9) voient leur temps d'éjaculation diminué avec le traitement, d'une valeur comprise entre 28% et 37%. Deux taureaux (taureaux 7 et 10) ne montrent pas d'amélioration sensible, et deux autres (taureaux 1 et 6) voient leur temps d'éjaculation diminué. Ces différences de comportement ne suffisent cependant pas à la mise en évidence d'une interaction « taureau / traitement ».

Pour 8 individus sur 10, le temps d'éjaculation maximal est diminué grâce à la composition C3. Le taureau 10 n'est pas affecté par le traitement, et le taureau 6 voit ce paramètre augmenté. Sur l'ensemble des 10 taureaux, on observe cependant que le traitement induit une diminution du temps d'éjaculation maximal moyen de 262 secondes, soit un gain de 18% par rapport aux témoins.

Les résultats ci-avant montrent clairement un effet individuel « taureau » largement significatif, et une absence d'interaction « taureau / traitement ». Ces mêmes résultats ont été obtenus sur tous les autres sites d'expérimentation, pour l'ensemble des compositions testées. Ceci tend à prouver que l'effet bénéfique du traitement sur le temps de réaction et sur le temps d'éjaculation est entièrement attribuable aux molécules conformes à l'invention testées.

La description ci-avant illustre clairement que par ses différentes caractéristiques et leurs avantages, la présente invention atteint les objectifs qu'elle s'était fixés. En particulier, elle fournit des compositions induisant une amélioration importante de la fonction reproductive chez le taureau. Des expérimentations réalisées ont en particulier démontré une amélioration importante de la libido des taureaux, l'utilisation des compositions conformes à l'invention permettant de réduire le temps de réaction d'environ 30 % et le temps d'éjaculation d'environ 20 %. De tels gains de temps auront des retentissements positifs notables sur l'organisation des collectes de semence, sur les coûts de production et la sécurité des taureliers au contact des animaux.

### BIBLIOGRAPHIE

Kumar KR, Archunan G, Jeyaraman R & Narasimhan S (2000) Chemical characterization of bovine urine with special reference to oestrus, Vet Res Commun, 24 (7), 445-454.
Sankar R & Archunan G (2004) Flehmen response in bull: role of vaginal mucus and other body fluids of bovine with special reference to estrus, Behav Processes, 67 (1), 81-86.
Sankar R & Archunan G (2008) Identification of putative pheromones in bovine (Bos taurus) faeces in relation to estrus détection, Anim Reprod Sci, 103 (1-2), 149-153.

## Revendications

1. Utilisation d'une composition comportant au moins une molécule volatile spécifique de l'oestrus chez la vache pour améliorer la fonction reproductive chez le taureau, **caractérisée en ce que** ladite molécule est choisie parmi le coumarin, le squalène, le 6-aminoundécane, la 2-butanone, l'acide 9-octadécénoïque et le 1,2-dichloroéthylène.

2. Utilisation d'une composition selon la revendication 1, pour améliorer la libido du taureau.

3. Utilisation d'une composition selon la revendication 1, pour améliorer la production de semence du taureau.

4. Utilisation selon l'une des revendications 1 à 3, **caractérisée en ce que** la composition est conditionnée sous forme de solution comportant au moins du glycérol en tant que solvant des molécules volatiles.

5. Utilisation selon l'une des revendications 1 à 4, **caractérisée en ce que** la concentration totale en molécules volatiles spécifiques de l'oestrus dans la composition est comprise entre 5 pg/mL et 100 pg/mL.

6. Utilisation selon l'une des revendications 1 à 4, **caractérisée en ce que** la concentration totale en molécules volatiles spécifiques de l'oestrus dans la composition est comprise entre 2,5 pg/mL et 5 pg/mL.

7. Utilisation selon l'une des revendications 1 à 4, **caractérisée en ce que** la concentration totale en molécules volatiles spécifiques de l'oestrus dans la composition est comprise entre 100 pg/mL et 500 pg/mL.

8. Utilisation selon l'une des revendications 1 à 5, **caractérisée en ce que** la concentration en molécules volatiles spécifiques de l'oestrus dans la composition est au moins égale à 25 pg/mL, +/- 5%.

9. Utilisation selon l'une des revendications 1 à 8, **caractérisée en ce que** la composition est maintenue à la périphérie des naseaux du taureau de manière à ce que ledit taureau puisse inhaler ladite composition.

10. Utilisation selon l'une des revendications 1 à 9, **caractérisée en ce que** la composition est conditionnée en spray de manière à pouvoir être pulvérisée dans les naseaux du taureau.

11. Utilisation selon l'une des revendications 1 à 10, **caractérisée en ce qu'**on procède à la saillie d'une femelle ou à la récolte de la semence directement après avoir soumis le taureau à ladite composition.

12. Utilisation selon l'une des revendications 1 à 11, **caractérisée en ce qu'**on soumet le taureau à ladite composition avant chaque récolte de semence ou avant chaque saillie.

13. Composition destinée à être utilisée sur un taureau, afin d'améliorer sa fonction reproductive, **caractérisée en ce qu'**elle contient au moins une molécule volatile spécifique de l'oestrus chez la vache choisie parmi le coumarin, le squalène, le 6-aminoundécane, la 2-butanone, l'acide 9-octadécénoïque et le 1,2-dichloroéthylène, la concentration totale en molécules volatiles spécifiques de l'oestrus de la vache dans ladite composition étant comprise entre 2,5 pg/mL et 500 pg/mL.

14. Composition selon la revendication 13, **caractérisée en ce qu'**elle contient du glycérol en tant que solvant des molécules volatiles.

15. Composition selon l'une des revendications 13 ou 14, **caractérisée en ce qu'**elle comporte les excipients nécessaires pour permettre une pulvérisation de ladite composition.

## Patentansprüche

1. Verwendung einer Zusammensetzung, enthaltend mindestens ein für die Brunft bei der Kuh spezifisches, flüchtiges Molekül, zur Verbesserung der Fortpflanzungsfähigkeit beim Bullen, **dadurch gekennzeichnet, dass** das Molekül aus Cumarin, Squalen, 6-Aminoundecan, 2-Butanon, 9-Octadecensäure und 1,2-Dichlorethylen ausgewählt wird.

2. Verwendung einer Zusammensetzung nach Anspruch 1 zur Verbesserung der Libido beim Bullen.

3. Verwendung einer Zusammensetzung nach Anspruch 1 zur Verbesserung der Samenproduktion beim Bullen.

4. Verwendung nach einem der Ansprüche 1 bis 3, **dadurch gekennzeichnet, dass** die Zusammensetzung in Form einer Lösung, die mindestens Glycerin als Lösungsmittel für die flüchtigen Moleküle enthält, zubereitet wird.

5. Verwendung nach einem der Ansprüche 1 bis 4, **dadurch gekennzeichnet, dass** die Gesamtkonzentration an für die Brunft spezifischen, flüchtigen Molekülen in der Zusammensetzung zwischen 5 pg/ml und 100 pg/ml beträgt.

6. Verwendung nach einem der Ansprüche 1 bis 4, **dadurch gekennzeichnet, dass** die Gesamtkonzentration an für die Brunft spezifischen, flüchtigen Molekülen in der Zusammensetzung zwischen 2,5 pg/ml und 5 pg/ml beträgt.

7. Verwendung nach einem der Ansprüche 1 bis 4, **dadurch gekennzeichnet, dass** die Gesamtkonzentration an für die Brunft spezifischen, flüchtigen Molekülen in der Zusammensetzung zwischen 100 pg/ml und 500 pg/ml beträgt.

8. Verwendung nach einem der Ansprüche 1 bis 5, **dadurch gekennzeichnet, dass** die Gesamtkonzentration an für die Brunft spezifischen, flüchtigen Molekülen in der Zusammensetzung mindestens gleich 25 pg/ml +/- 5% ist.

9. Verwendung nach einem der Ansprüche 1 bis 8, **dadurch gekennzeichnet, dass** die Zusammensetzung an der Peripherie der Nasenlöcher des Bullen derart gehalten wird, dass der Bulle die Zusammensetzung inhalieren kann.

10. Verwendung nach einem der Ansprüche 1 bis 9, **dadurch gekennzeichnet, dass** die Zusammensetzung zu einem Spray zubereitet wird, derart, dass sie in die Nasenlöcher des Bullen zerstäubt werden kann.

11. Verwendung nach einem der Ansprüche 1 bis 10, **dadurch gekennzeichnet, dass** man das Decken des Weibchens oder die Ernte des Samens durchführt, direkt nachdem man den Bullen der Zusammensetzung ausgesetzt hat.

12. Verwendung nach einem der Ansprüche 1 bis 11, **dadurch gekennzeichnet, dass** man den Bullen der Zusammensetzung vor jeder Ernte von Samen oder vor jedem Decken aussetzt.

13. Zusammensetzung für die Verwendung bei einem Bullen zur Verbesserung von dessen Fortpflanzungsfähigkeit, **dadurch gekennzeichnet, dass** sie mindestens ein für die Brunft bei der Kuh spezifisches, flüchtiges Molekül enthält, ausgewählt aus Cumarin, Squalen, 6-Aminoundecan, 2-Butanon, 9-Octadecensäure und 1,2-Dichlorethylen, wobei die Gesamtkonzentration an für die Brunft bei der Kuh spezifischen, flüchtigen Molekülen in der Zusammensetzung zwischen 2,5 pg/ml und 500 pg/ml beträgt.

14. Zusammensetzung nach Anspruch 13, **dadurch gekennzeichnet, dass** sie Glycerin als Lösungsmittel für die flüchtigen Moleküle enthält.

15. Zusammensetzung nach einem der Ansprüche 13 oder 14, **dadurch gekennzeichnet, dass** sie die zur Ermöglichung der Zerstäubung der Zusammensetzung benötigten Exzipienten enthält.

## Claims

1. Use of a composition comprising at least one volatile molecule specific to the estrus of cows in order to improve the reproductive function of a bull, **characterized in that** said molecule is selected from among coumarin, squalene, 6-amino undecane, 2-butanone, 9-octadecenoic acid and 1,2-dichloroethylene.

2. Use of a composition according to claim 1, in order to improve the bull's libido.

3. Use of a composition according to claim 1, in order to improve the production of semen by the bull.

4. Use according to one of claims 1 to 3, **characterized in that** the composition is presented in the form of a solution comprising at least glycerol as a solvent of the volatile molecules.

5. Use according to one of claims 1 to 4, **characterized in that** the total concentration of volatile molecules specific to estrus in the composition is between 5 pg/mL and 100 pg/mL.

6. Use according to one of claims 1 to 4, **characterized in that** the total concentration of volatile molecules specific to the estrus in the composition is between 2.5 pg/mL and 5 pg/mL.

7. Use according to one of claims 1 to 4, **characterized in that** the total concentration of volatile molecules specific to the estrus in the composition is between 100 pg/mL and 500 pg/mL.

8. Use according to one of claims 1 to 5, **characterized in that** the concentration of volatile molecules specific to the estrus in the composition is at least equal to 25 pg/mL, +/- 5%.

9. Use according to one of claims 1 to 8, **characterized in that** the composition is kept at the periphery of the bull's nostrils so that said bull can inhale said composition.

10. Use according to one of claims 1 to 9, **characterized in that** the composition is presented as a spray so it can be sprayed into the bull's nostrils.

11. Use according to one of claims 1 to 10, **characterized in that** serving a female or collecting the semen takes place directly after said composition has been presented to the bull.

12. Use according to one of claims 1 to 11, **characterized in that** said composition is presented to the bull before each semen collection or before each covering.

13. Composition intended for use on a bull, in order to improve its reproductive function, **characterized in that** it comprises at least one volatile molecule specific to the estrus of cows selected from among coumarin, squalene, 6-amino undecane, 2-butanone, 9-octadecenoic acid and 1,2-dichloroethylene, the total concentration of volatile molecules specific to the estrus of cows being between 2.5 pg/mL and 500 pg/mL.

14. Composition according to claim 13, **characterized in that** it contains glycerol as a solvent of the volatile molecules.

15. Composition according to one of claims 13 or 14, **characterized in that** it comprises the excipients necessary to enable said composition to be sprayed.
